# DEMANDE DE BREVET EUROPEEN

(11) **EP 1 428 516 A1**
(43) Date de publication de la demande: **16.06.2004**
(21) Numéro de dépôt: 03293141.2
(22) Date de dépôt: 12.12.2003
(51) Int. Cl.: A61K 7/13

(54) **Composition tinctoriale comprenant une paraphénylènediamine tertiaire cationique contenant un noyau pyrrolidinique et un ester de polyol; procédés et utilisations**

(30) Priorité: 13.12.2002 FR 0215776
(71) Demandeur: L'OREAL, 75008 Paris (FR)
(72) Inventeur: Cotteret, Jean, 78480 Verneuil sur Seine (FR); Lagrange, Alain, 77700 Coupvray (FR)
(74) Mandataire: Dossmann, Gérard

(57) **Abrégé**

La présente demande a pour objet une composition tinctoriale pour la teinture des fibres kératiniques, en particulier des fibres kératiniques humaines telles que les cheveux, comprenant dans un milieu de teinture approprié au moins une paraphénylènediamine tertiaire cationique contenant un noyau pyrrolidinique et au moins un ester de polyol.

L'invention a aussi pour objet le procédé de teinture mettant en oeuvre cette composition.

## Description

La présente demande a pour objet une composition tinctoriale pour la teinture des fibres kératiniques , en particulier des fibres kératiniques humaines telles que les cheveux, comprenant dans un milieu de teinture approprié au moins une paraphénylènediamine tertiaire cationique contenant un noyau pyrrolidinique, et au moins un ester de polyol.

L'invention a aussi pour objet l'utilisation de cette composition pour la teinture des fibres kératiniques ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il a déjà été proposé dans la demande de brevet WO 02/45675 d'utiliser des compositions de teintures d'oxydation des fibres kératiniques comprenant une paraphénylènediamine tertiaire cationique contenant un noyau pyrrolidinique.

Ces paraphénylènediamines tertiaires cationiques contenant un noyau pyrrolidinique conduisent à des compositions qui présentent une innocuité généralement considérée comme meilleure que les compositions contenant des paraphénylènediamines classiques. Cependant les nuances obtenues lorsqu'on utilise ces compositions sont nettement moins puissantes et nettement plus sélectives, c'est-à-dire que les teintures obtenues présentent des variations de colorations importantes en fonction du degré de sensibilisation des différents cheveux ou des différentes zones d'un même cheveu. La ténacité de ces nuances peut aussi varier fortement suivant le degré de sensibilisation. En outre, les colorations obtenues sont également souvent plus grises, c'est-à-dire moins chromatiques.

De manière surprenante et avantageuse, la Demanderesse vient de découvrir qu'il est possible d'obtenir de nouvelles compositions pour la teinture des fibres kératiniques, en particulier des fibres kératiniques humaines telles que les cheveux, capables de pallier les inconvénients cités ci-dessus, et notamment de conduire à des colorations aux nuances variées, chromatiques, puissantes, esthétiques, peu sélectives et résistant bien aux diverses agressions que peuvent subir les fibres, en associant au sein d'une composition, au moins une paraphénylènediamine tertiaire cationique contenant un noyau pyrrolidinique et au moins un ester de polyol. En outre, ces compositions présentent un bon profil toxicologique.

L'invention a aussi pour objet un procédé de teinture mettant en oeuvre cette composition ainsi qu'un dispositif à plusieurs compartiments ou "kit" de teinture.

Un autre objet de l'invention est l'utilisation de la composition de la présente invention pour la teinture des fibres kératiniques, en particulier les fibres kératiniques humaines telles que les cheveux.

La composition de la présente invention permet en particulier d'obtenir une coloration de fibres kératiniques chromatique, très puissante, peu sélective et tenace tout en évitant la dégradation de ces fibres.

Au sens de la présente invention, on entend par paraphénylènediamine tertiaire cationique contenant un noyau pyrrolidinique, une paraphénylènediamine possédant un groupement NH₂ et en para de celui-ci une fonction amine di-substituée dont les substitutions forment avec l'atome d'azote un noyau pyrrolidinique, la molécule possédant au moins un atome d'azote quaternisé.

Dans le cadre de la présente invention, on entend par alkyle, des radicaux linéaires ou ramifiés par exemple méthyle, éthyle, n-propyle, iso-propyle, butyle, etc. Un radical alcoxy est un radical alk-O, le radical alkyle ayant la définition donnée ci dessus. Halogène désigne de préférence Cl, Br, I, F.

Parmi les paraphénylènediamines tertiaires cationiques contenant un noyau pyrrolidinique utilisables dans la composition selon la présente invention, on peut notamment citer les composés de formule (I) suivante et leur sels d'addition 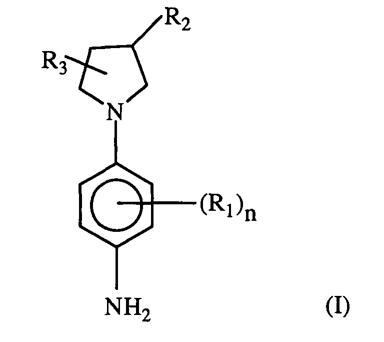 dans laquelle
- n varie de 0 à 4, étant entendu que lorsque n est supérieur ou égal à 2 alors les radicaux R₁ peuvent être identiques ou différents,
- R₁ représente un atome d'halogène ; une chaîne hydrocarbonée en C₁-C₆, aliphatique ou alicyclique, saturée ou insaturée, la chaîne pouvant être interrompue par un ou plusieurs atomes d'oxygène, d'azote, de silicium, de soufre ou par un groupement SO₂, et pouvant être substituée par un ou plusieurs radicaux hydroxy ou amino ; un radical onium Z, le radical R₁ ne comportant pas de liaison peroxyde, ni de radicaux diazo, nitro ou nitroso ,
R₂ représente un radical onium Z ou un radical -X-C=NR₈-NR₉R₁₀ dans lequel X représente un atome d'oxygène ou un radical -NR₁₁ et R₈, R₉, R₁₀ et R₁₁ représentent un atome d'hydrogène, un radical alkyle en C₁-C₄ ou un radical hydroxyalkyle en C₁-C₄,
R₃ représente un atome d'hydrogène ou un radical hydroxy.

Onium désigne un radical quaternaire d'une base azotée.

A titre d'exemple, R1 peut être un atome de chlore, un radical méthyle, éthyle, isopropyle, vinyle, allyle, méthoxyméthyle, hydroxyéthyle, 1-carboxyméthyle, 1-aminométhyle, 2-carboxyéthyle, 2-hydroxyéthyle, 3-hydroxypropyle, 1,2-dihydroxyéthyle, 1-hydroxy-2-aminoéthyle, 1-amino-2-hydroxyéthyle, 1,2- diaminoéthyle, méthoxy, éthoxy, allyloxy, 2-hydroxyéthyloxy.

En particulier, n est égal à 0.

Dans la formule (I), lorsque n est égal à 1, R1 est de préférence un atome d'halogène ; une chaîne hydrocarbonée en C₁-C₆, aliphatique ou alicyclique, saturée ou insaturée, un ou plusieurs atomes de carbone pouvant être remplacés par un atome d'oxygène, d'azote, de silicium, de soufre, par un groupement SO₂, le radical R₁ ne comportant pas de liaison peroxyde, ni de radicaux diazo, nitro ou nitroso. De préférence, R1 est choisi parmi le chlore, le brome, un radical alkyle en C₁-C₄, hydroxyalkyle en C₁-C₄, aminoalkyle en C₁-C₄, alcoxy en C₁-C₄, hydroxyalcoxy en C₁-C₄. A titre d'exemple, R1 est choisi parmi un radical méthyle, hydroxyméthyle, 2-hydroxyéthyle, 1,2-dihydroxyéthyle, méthoxy, isopropyloxy, 2-hydroxyéthoxy.

Le radical R₂ de la formule (I) est selon un mode de réalisation particulier le radical onium Z correspondant à la formule (II) 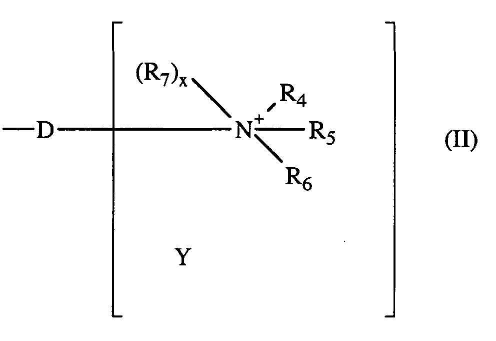 dans laquelle
- D est une simple liaison ou une chaîne alkylène en C₁-C₁₄, linéaire ou ramifiée pouvant être interrompue par un ou plusieurs hétéroatomes choisis parmi l'oxygène, le soufre ou l'azote, et pouvant être substituée par un ou plusieurs radicaux hydroxyle, alcoxy en C₁-C₆ ou amino, et pouvant porter une ou plusieurs fonctions cétone ;
- R₄, R₅ et R₆, pris séparément, représentent un radical alkyle en C₁-C₁₅ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical amidoalkyle en C₁-C₆ ; un radical trialkyl(C₁-C₆)silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl(C₁-C₆)carbonyle, amido ou alkyl(C₁-C₆)sulfonyle ; ou
- R₄, R₅ et R₆ ensemble, deux à deux, forment, avec l'atome d'azote auxquels ils sont rattachés, un cycle saturé carboné à 4, 5, 6 ou 7 chaînons pouvant contenir un ou plusieurs hétéroatomes tel que par exemple un cycle azétidine, un cycle pyrrolidine, un cycle pipéridine, un cycle pipérazine ou un cycle morpholine, le cycle cationique pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical carboxyle, un radical alkyl(C₁-C₆)carbonyl, un radical thio (-SH), un radical thioalkyle (-R-SH) en C₁-C₆, un radical alkyl(C₁-C₆)thio, un radical amino, un radical amino mono ou disubstitué par un radical alkyl(C₁-C₆), alkyl(C₁-C₆)carbonyle, amido ou alkyl(C₁-C₆)sulfonyle ;
- R₇ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆ ; un radical aminoalkyle en C₁-C₆ dont l'amine est mono- ou di-subsituée par un radical alkyl(C₁-C₆), alkyl(C₁-C₆)carbonyle, amido ou alkyl(C₁-C₆)sulfonyle ; un radical carboxyalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆; un radical alkyl(C₁-C₆)sulfonylalkyle en C₁-C₆; un radical alkyl(C₁-C₆)carbonylalkyle en C₁-C₆; un radical N-alkyl(C₁-C₆)carbamylalkyle en C₁-C₆; un radical N-alkyl(C₁-C₆)sulfonamidoalkyle en C₁-C₆;
- x est 0 ou 1,
   - lorsque x = 0, alors le bras de liaison est rattaché à l'atome d'azote portant les radicaux R₄ à R₆,
   - lorsque x = 1, alors deux des radicaux R₄ à R₆ forment conjointement avec l'atome d'azote auquel ils sont rattachés un cycle saturé à 4, 5, 6 ou 7 chaînons et D est lié à un atome de carbone du cycle saturé ;
- Y est un contre ion.

Dans la formule (II), lorsque x est égal à 0, alors R₄, R₅ et R₆ séparément sont choisis de préférence parmi un radical alkyle en C₁-C₆, un radical monohydroxyalkyle en C₁-C₄, un radical polyhydroxyalkyle en C₂-C₄, un radical alcoxy(C₁-C₆)alkyle en C₁-C₄, un radical amidoalkyle en C₁-C₆, un radical trialkyl(C₁-C₆)silanealkyle en C₁-C₆, ou R₄ avec R₅ forment ensemble un cycle azétidine, un cycle pyrrolidine, pipéridine, pipérazine, morpholine, R₆ étant choisi dans ce cas parmi un radical alkyle en C₁-C₆; un radical monohydroxyalkyle en C₁-C₆; un radical polyhydroxyalkyle en C₂-C₆; un radical aminoalkyle en C₁-C₆, un radical aminoalkyle mono ou disubstitué par un radical alkyl(C₁-C₆), alkyl(C₁-C₆)carbonyle, amido ou alkyl(C₁-C₆)sulfonyle ; un radical carbamylalkyle en C₁-C₆; un radical trialkyl(C₁-C₆)silanealkyle en C₁-C₆; un radical alkyl(C₁-C₆)carboxyalkyle en C₁-C₆; un radical alkyl(C₁-C₆)carbonyalkyle en C₁-C₆; un radical N-alkyl(C₁-C₆)carbamylalkyle en C₁-C₆.

Lorsque x est égal à 1, alors R₇ est de préférence choisi parmi un radical alkyle en C₁-C₆; un radical monohydroxyalkyle en C₁-C₆; un radical polyhydroxyalkyle en C₂-C₆; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, amido ou alkyl(C₁-C₆)sulfonyle ; un radical carbamylalkyle en C₁-C₆; un radical trialkyl(C₁-C₆)silanealkyle en C₁-C₆ ; un radical alkyl(C₁-C₆)carboxyalkyle en C₁-C₆; un radical alkyl(C₁-C₆)carbonylalkyle en C₁-C₆ ; un radical N-alkyl(C₁-C₆)carbamylalkyle en C₁-C₆; R₄ avec R₅ ensemble forment un cycle azétidine, pyrrolidine, pipéridine, pipérazine, morpholine, R₆ étant choisi dans ce cas parmi un radical alkyle en C₁-C₆; un radical monohydroxyalkyle en C₁-C₆; un radical polyhydroxyalkyle en C₂-C₆; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, amido ou alkyl(C₁-C₆)sulfonyle ; un radical carbamylalkyle en C₁-C₆ ; un radical trialkyl(C₁-C₆)silanealkyle en C₁-C₆; un radical alkyl(C₁-C₆)carboxyalkyle en C₁-C₆; un radical alkyl(C₁-C₆)carbonylalkyle en C₁-C₆ ; un radical N-alkyl(C₁-C₆)carbamylalkyle en C₁-C₆.

Dans la formule (II), D est de préférence une simple liaison ou une chaîne alkylène pouvant être substituée.

Lorsque le radical R₂ correspond à la formule (II), il est de préférence un radical trialkylammonium dont les radicaux alkyles peuvent être substitués.

Selon un deuxième mode de réalisation, le radical R₂ représente le radical onium Z correspondant à la formule (III) 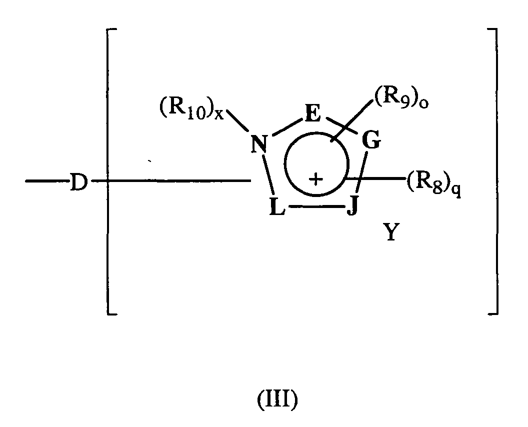 dans laquelle
- D est une simple liaison ou une chaîne alkylène en C₁-C₁₄, linéaire ou ramifiée pouvant être interrompue par un ou plusieurs hétéroatomes choisis parmi l'oxygène, le soufre ou l'azote, et pouvant être substituée par un ou plusieurs radicaux hydroxyle, alcoxy en C₁-C₆ ou amino, et pouvant porter une ou plusieurs fonctions cétone;
- les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique,
- q est un nombre entier compris entre 0 et 4 inclus ;
- o est un nombre entier compris entre 0 et 3 inclus ;
- q+o est un nombre entier compris entre 0 et 4 ;
- les radicaux R₈, identiques ou différents, représentent un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical carboxyle, un radical alkylcarbonyle en C₁-C₆, un radical thio, un radical thioalkyle en C₁-C₆, un radical alkyl(C₁-C₆)thio, un radical amino, un radical amino mono- ou di-substitué par un radical alkyl(C₁-C₆), alkyl(C1-C6)carbonyle, amido, alkyl(C₁-C₆)sulfonyle ; un radical monohydroxyalkyle en C₁-C₆ ou un radical polyhydroxyalkyle en C₂-C₆ ; étant entendu que les radicaux R₈ sont portés par un atome de carbone,
- les radicaux R₉, identiques ou différents, représentent un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; étant entendu que les radicaux R₉ sont portés par un azote,
- R₁₀ représente un radical alkyle en C₁-C₆; un radical monohydroxyalkyle en C₁-C₆; un radical polyhydroxyalkyle en C₂-C₆; un radical aryle ; un radical benzyle ; un radical aminoalkyle en C₁-C₆, un radical aminoalkyle en C₁-C₆dont l'amine est substituée par un radical alkyl(C₁-C₆), alkyl(C₁-C₆)carbonyle, amido ou alkyl(C₁-C₆)sulfonyle ; un radical carboxyalkyle en C₁-C₆; un radical carbamylalkyle en C₁-C₆; un radical trifluoroalkyle en C₁-C₆; un radical trialkyl(C₁-C₆)silanealkyle en C₁-C₆; un radical sulfonamidoalkyle en C₁-C₆; un radical alkyl(C₁-C₆)carboxyalkyle en C₁-C₆ ; un radical alkyl(C₁-C₆)sulfinylalkyle en C₁-C₆; un radical alkyl(C₁-C₆)sulfonylalkyle en C₁-C₆ ; un radical alkyl(C₁-C₆)carbonylalkyle en C₁-C₆; un radical N-alkyl(C₁-C₆)carbamylalkyle en C₁-C₆; un radical N-alkyl(C₁-C₆)sulfonamidoalkyle en C₁-C₆;
- x est 0 ou 1
   - lorsque x = 0, le bras de liaison D est rattaché à l'atome d'azote,
   - lorsque x = 1, le bras de liaison D est rattaché à l'un des sommets E, G, J ou L,
- Y est un contre-ion.

Les sommets E, G, J et L forment de préférence un cycle imidazolique.

Parmi les radicaux R₂ de formules (III), on préfère les radicaux dans lesquels x est égal à 0, D est une simple liaison ou une chaîne alkylène pouvant être substituée.

Selon un troisième mode de réalisation, R2 représente le radical onium Z correspondant à la formule (IV) 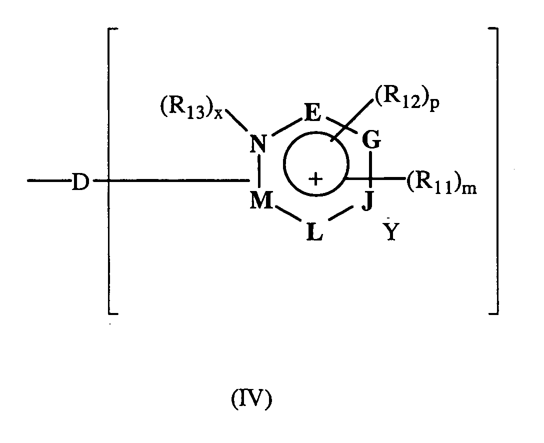 dans laquelle :
- D est une simple liaison ou une chaîne alkylène en C₁-C₁₄, linéaire ou ramifiée pouvant être interrompue par un ou plusieurs hétéroatomes choisis parmi un atome d'oxygène, de soufre ou d'azote, et pouvant être substituée par un ou plusieurs radicaux hydroxyle, alcoxy en C₁-C₆ ou amino, et pouvant porter une ou plusieurs fonctions cétone;
- les sommets E, G, J, L et M identiques ou différents, représentent un atome de carbone, d'oxygène, de soufre ou d'azote pour former un cycle choisi parmi les cycles pyridiniques, pyrimidiniques, pyraziniques, triaziniques et pyridaziniques ;
- p est un nombre entier compris entre 0 et 3 inclus ;
- m est un nombre entier compris entre 0 et 5 inclus ;
- p+m est un nombre entier compris entre 0 et 5 ;
- les radicaux R₁₁, identiques ou différents, représentent un atome d'halogène, un radical hydroxyle, un radical alkyle en C₁-C₆, un radical monohydroxyalkyle en C₁-C₆, un radical polyhydroxyalkyle en C2-C6, un radical alcoxy en C₁-C₆, un radical trialkyl(C₁-C₆)silanealkyle en C₁-C₆, un radical amido, un radical carboxyle, un radical alkylcarbonyle en C₁-C₆, un radical thio, un radical thioalkyle en C₁-C₆, un radical alkyl(C₁-C₆)thio, un radical amino, un radical amino substitué par un radical alkyl(C₁-C₆), alkyl(C₁-C₆)carbonyle, amido ou alkyl(C₁-C₆)sulfonyle ; un radical monohydroxyalkyle en C₁-C₆ou un radical polyhydroxyalkyle en C₂-C₆; étant entendu que les radicaux R₁₁ sont portés par un atome de carbone,
- les radicaux R₁₂ , identiques ou différents, représentent un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; étant entendu que les radicaux R₁₂ sont portés par un azote,
- R₁₃ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est mono- ou di-substituée par un radical alkyl(C₁-C₆), alkyl(C₁-C₆)carbonyle, amido ou alkyl(C₁-C₆)sulfonyle ; un radical carboxyalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- x est 0 ou 1
   - lorsque x = 0, le bras de liaison D est rattaché à l'atome d'azote,
   - lorsque x = 1, le bras de liaison D est rattaché à l'un des sommets E, G, J, L ou M,
- Y représente un contre ion.

De préférence, les sommets E, G, J, L et M forment avec l'azote du cycle un cycle pyridinique et pyrimidinique.

Lorsque x est égal à 0 alors R₁₁ est de préférence choisi parmi un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical alkylcarbonyle en C₁-C₆, un radical amino, un radical amino mono- ou di-substitué par un radical alkyl(C₁-C₆), alkyl(C₁-C₆)carbonyle, amido ou alkyl(C₁-C₆)sulfonyle ; un radical monohydroxyalkyle en C₁-C₆ ou un radical polyhydroxyalkyle en C₂-C₆ et R₁₂ est choisi parmi un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆.

Lorsque x est égal à 1, R₁₃ est de préférence choisi parmi un radical alkyle en C₁-C₆ ; un radical monohydroxyalkyle en C₁-C₆ ; un radical polyhydroxyalkyle en C₂-C₆ ; un radical aminoalkyle en C₁-C₆, un radical aminoalkyle en C₁-C₆ dont l'amine est mono- ou di-substituée par un radical alkyl(C₁-C₆), alkyl(C₁-C₆)carbonyle, un radical amido ou un radical alkyl(C₁-C₆)sulfonyle ; un radical carbamylalkyle en C₁-C₆ ; un radical trialkyl(C₁-C₆)silanealkyle en C₁-C₆ ; un radical alkyl(C₁-C₆)carbonylalkyle en C₁-C₆ ; un radical N-alkyl(C₁-C₆)carbamylalkyle en C₁-C₆ ; R₁₁ est choisi parmi un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical alkylcarbonyle en C₁-C₆, un radical amino, un radical amino mono- ou di- substitué par un radical alkyl(C₁-C₆), alkyl(C₁-C₆)carbonyle, amido ou alkyl(C₁-C₆)sulfonyle ; et R₁₂ est choisi parmi un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆.

De préférence, R₁₁, R₁₂ et R₁₃ sont des radicaux alkyles pouvant être substitués.

Le radical R₂ peut aussi représenter un radical onium de formule

-XP(O)(O-)OCH₂CH₂N⁺(CH₃)₃

où X représente un atome d'oxygène ou un radical-NR₁₄, R₁₄ représentant un hydrogène, un radical alkyle en C₁-C₄ ou un radical hydroxyalkyle.

Dans le cadre de l'invention, R₂ peut aussi représenter un radical guanidine de formule -X-C=NR₈-NR₉R₁₀, X représente un atome d'oxygène ou un radical -NR₁₁, R₈, R₉, R₁₀ et R₁₁ représentant un hydrogène, un radical alkyle en C₁-C₄ ou un radical hydroxyalkyle. Selon un mode de réalisation particulier, X est -NR₁₁, R₈ est un hydrogène, R₉ et R₁₀ sont choisis parmi l'hydrogène ou un radical alkyle, de préférence méthyle.

Le pKa du radical guanidine R₂ est en général tel que ce substituant est présent sous forme cationique (=NR₈H+) dans les conditions classiques de teinture des cheveux par oxydation.

Dans le cadre de l'invention, le contre ion peut être issu d'un atome d'halogène tel que le brome, le chlore, le fluor ou l'iode, d'un hydroxyde, d'un citrate, d'un succinate, d'un tartrate, d'un lactate, d'un tosylate, d'un mésylate, d'un benzènesulfonate, d'un acétate, d'un hydrogènesulfate ou d'un alkylsulfate en C₁-C₆ tel que par exemple le méthylsulfate, ou l'éthylsulfate.

Dans le cadre de la présente demande, on utilise de manière préférée les paraphénylènediamines tertiaires cationiques contenant un noyau pyrrolidinique décrites ci-dessus et pour lesquelles R₂ est de formule II ou III. De manière encore plus préférée on utilise les paraphénylènediamines tertiaires cationiques contenant un noyau pyrrolidinique décrites ci-dessus pour lesquelles R₂ est de formule II ou de formule III, avec x=0 et pour lesquelles n=0.

A titre d'exemples de dérivés de formule (I), on peut citer :

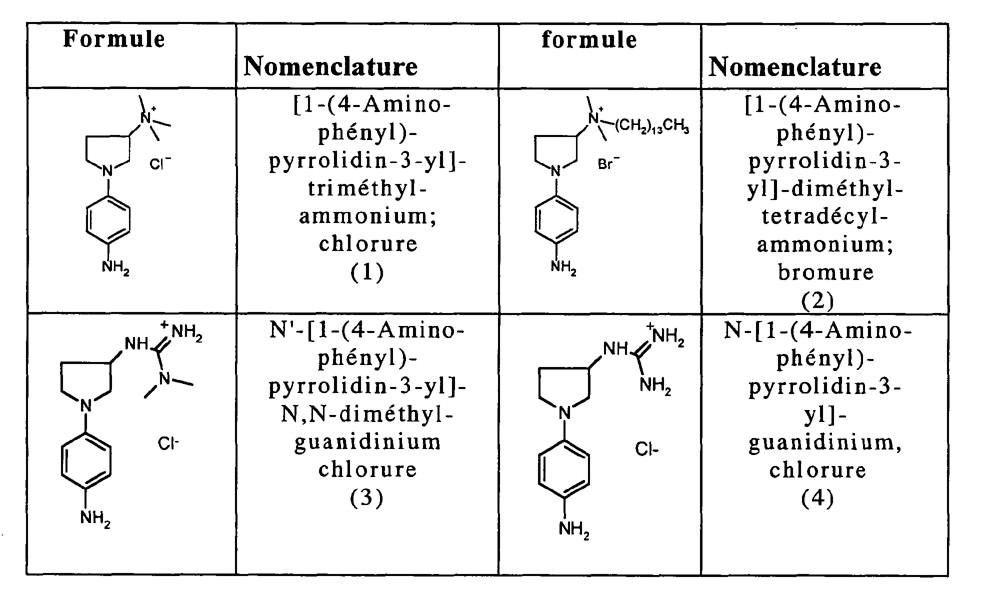

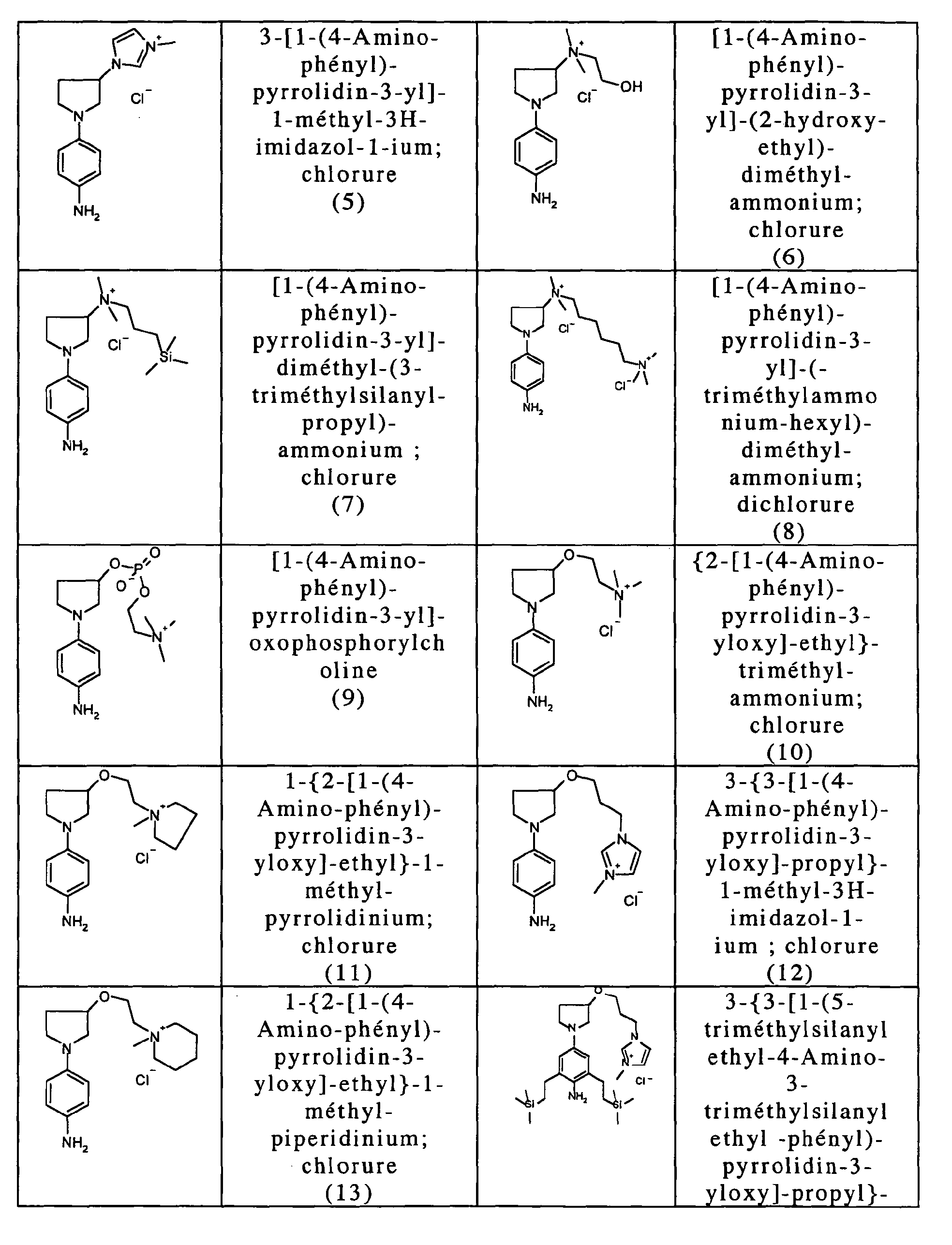

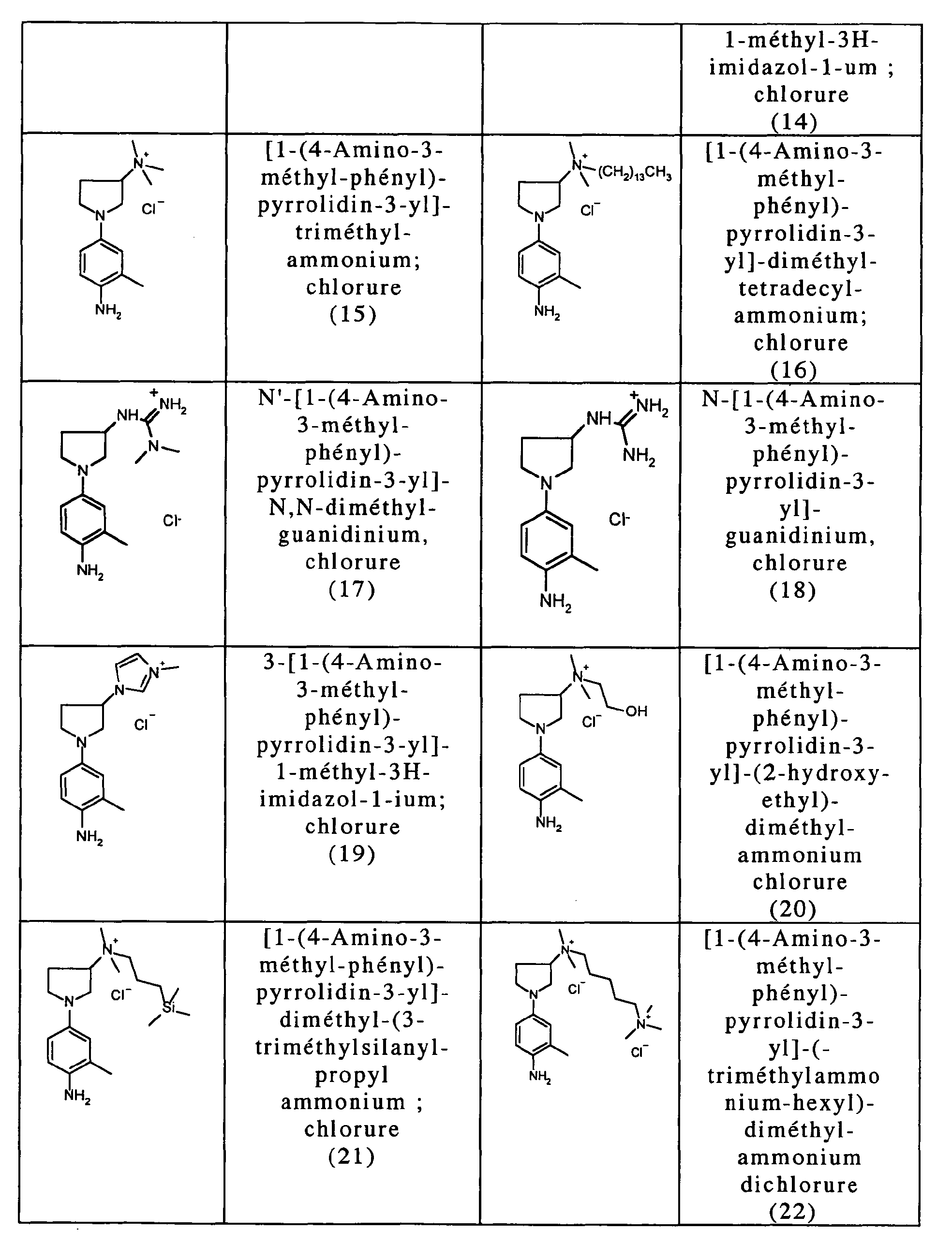

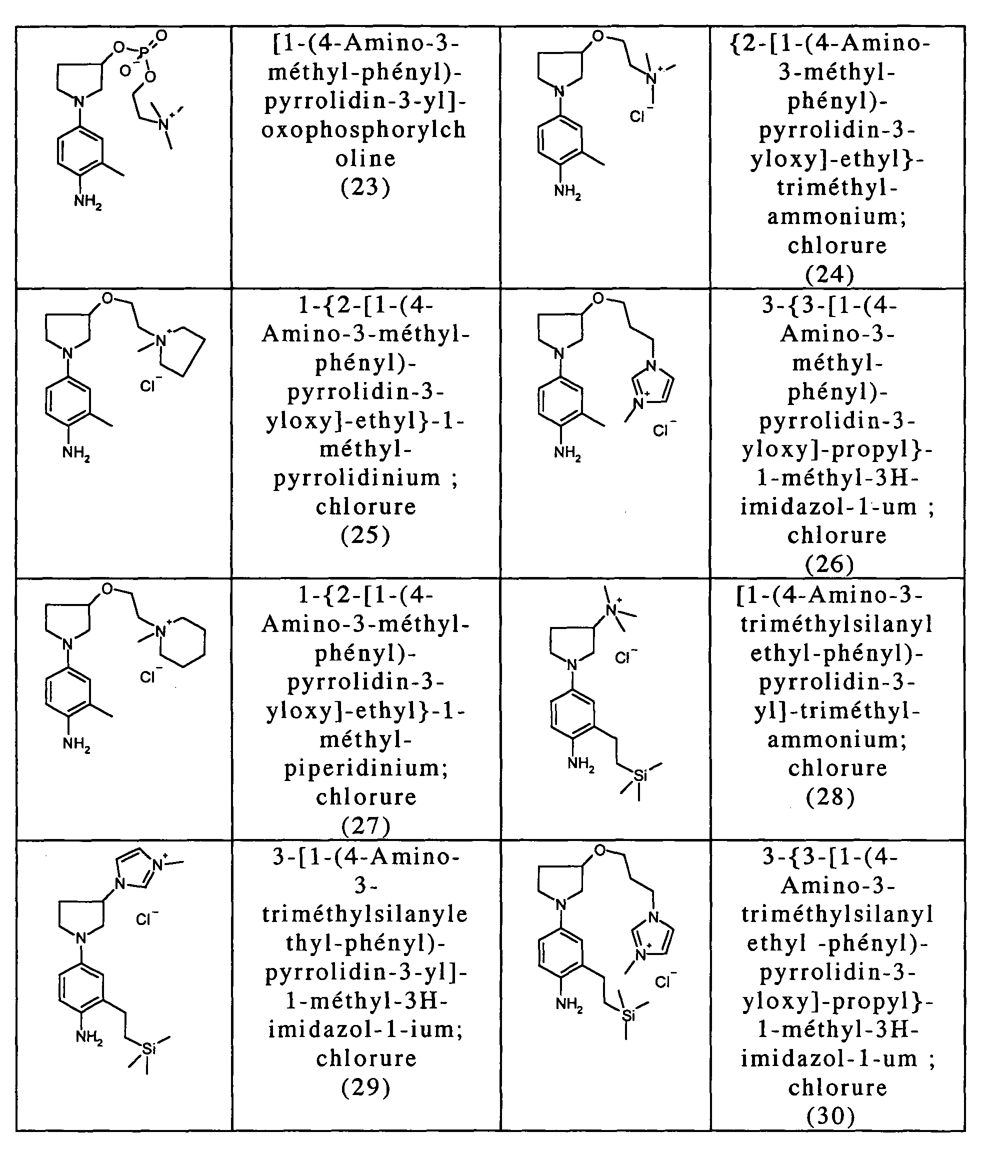

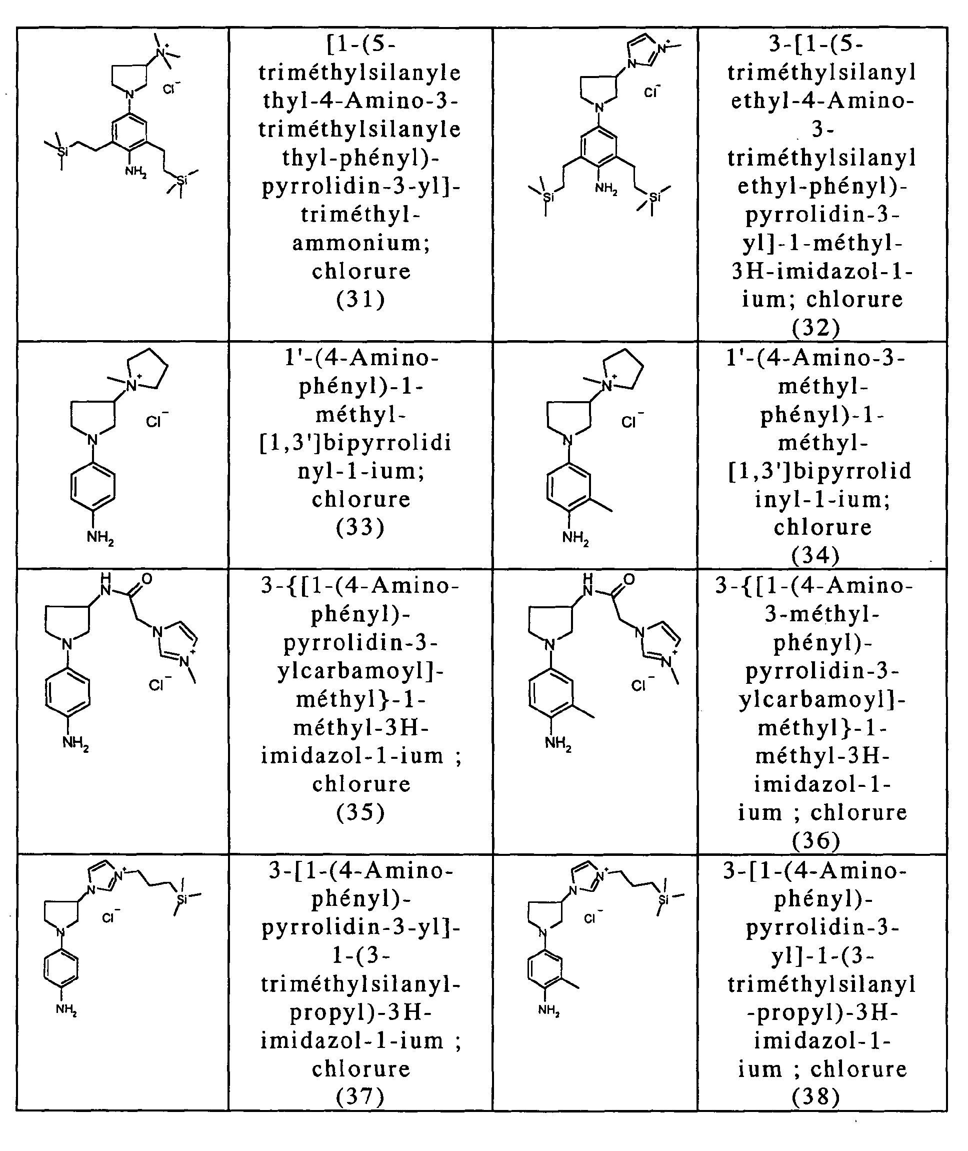

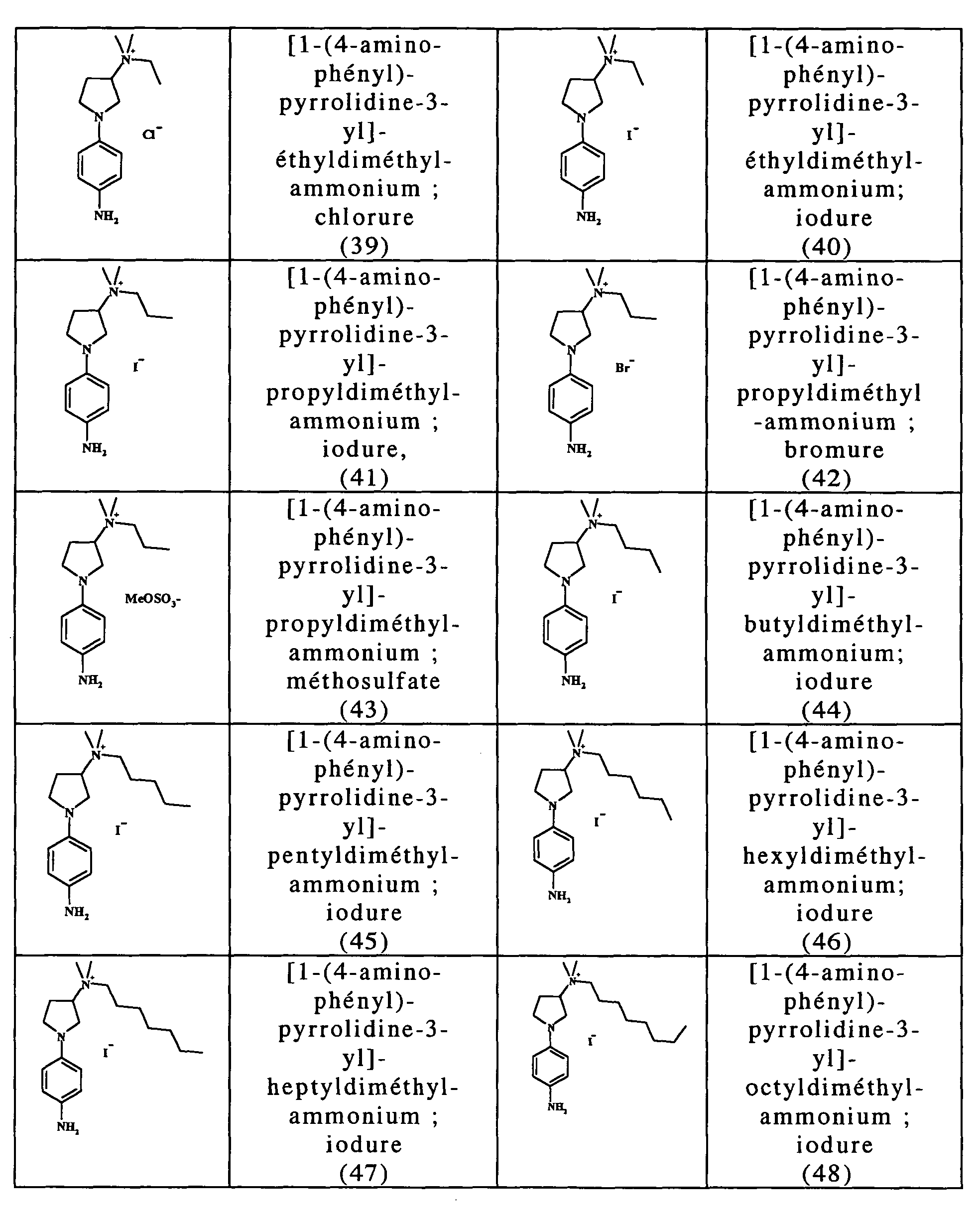

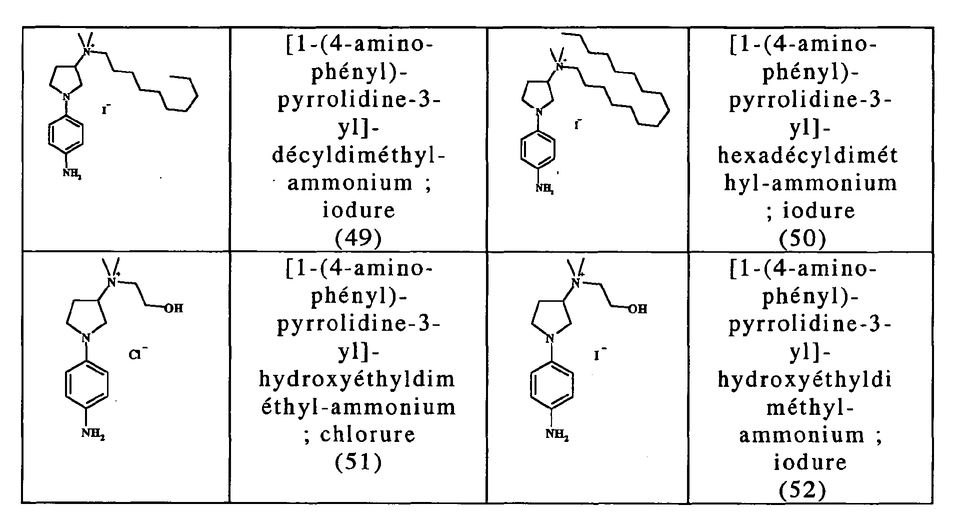

Les dérivés de formule I utilisés de manière préférée sont les :
[1-(4-Amino-phényl)-pyrrolidin-3-yl]-triméthyl-ammonium; chlorure ;
[1-(4-Amino-phényl)-pyrrolidin-3-yl]-diméthyl-tetradécyl-ammonium; bromure ;
N'-[1-(4-Amino-phényl)-pyrrolidin-3-yl]-N,N-diméthyl-guanidinium chlorure
N-[1-(4-Amino-phényl)-pyrrolidin-3-yl]- guanidinium chlorure
3-[1-(4-Amino-phényl)-pyrrolidin-3-yl]-1-méthyl-3H-imidazol-1-ium; chlorure ;
[1-(4-Amino-phényl)-pyrrolidin-3-yl]-(2-hydroxy-ethyl)-diméthyl-ammonium; chlorure
[1-(4-Amino-phényl)-pyrrolidin-3-yl]-diméthyl-(3-triméthylsilanyl-propyl)-ammonium ; chlorure ;
[1-(4-Amino-3-méthyl-phényl)-pyrrolidin-3-yl]-triméthyl-ammonium; chlorure
[1-(4-Amino-3-méthyl-phényl)-pyrrolidin-3-yl]-diméthyl-tetradecyl-ammonium; chlorure
N'-[1-(4-Amino-3-méthyl-phényl)-pyrrolidin-3-yl]-N,N-diméthyl- guanidinium chlorure
N-[1-(4-Amino-3-méthyl-phényl)-pyrrolidin-3-yl]- guanidinium chlorure
3-[1-(4-Amino-3-méthyl-phényl)-pyrrolidin-3-yl]-1-méthyl-3H-imidazol-1-ium; chlorure
[1-(4-Amino-3-méthyl-phényl)-pyrrolidin-3-yl]-(2-hydroxy-ethyl)-diméthyl-ammonium chlorure
[1-(4-Amino-3-méthyl-phényl)-pyrrolidin-3-yl]-diméthyl-(3-triméthylsilanyl-propyl ammonium ; chlorure
1'-(4-Amino-phényl)-1-méthyl-[1,3']bipyrrolidinyl-1-ium; chlorure
1'-(4-Amino-3-méthyl-phényl)-1-méthyl-[1,3']bipyrrolidinyl-1-ium; chlorure
3-{[1-(4-Amino-phényl)-pyrrolidin-3-ylcarbamoyl]-méthyl}-1-méthyl-3H-imidazol-1-ium ; chlorure
3-{[1-(4-Amino-3-méthyl-phényl)-pyrrolidin-3-ylcarbamoyl]-méthyl}-1-méthyl-3H- imidazol-1-ium ; chlorure
3-[1-(4-Amino-phényl)-pyrrolidin-3-yl]-1-(3-triméthylsilanyl-propyl)-3H-imidazol-1-ium ; chlorure
3-[1-(4-Amino-3-méthyl-phényl)-pyrrolidin-3-yl]-1-(3-triméthylsilanyl-propyl)-3H-imidazol-1-ium ; chlorure
[1-(4-amino-phényl)-pyrrolidine-3-yl]-éthyldiméthyl-ammonium ; chlorure
[1-(4-amino-phényl)-pyrrolidine-3-yl]-éthyldiméthyl-ammonium ; iodure
[1-(4-amino-phényl)-pyrrolidine-3-yl]-propyldiméthyl-ammonium ; iodure,
[1-(4-amino-phényl)-pyrrolidine-3-yl]-propyldiméthyl-ammonium ; bromure
[1-(4-amino-phényl)-pyrrolidine-3-yl]-propyldiméthyl-ammonium ; méthosulfate
[1-(4-amino-phényl)-pyrrolidine-3-yl]-butyldiméthyl-ammonium ; iodure
[1-(4-amino-phényl)-pyrrolidine-3-yl]-pentyldiméthyl-ammonium ; iodure
[1-(4-amino-phényl)-pyrrolidine-3-yl]-hexyldiméthyl-ammonium ; iodure
[1-(4-amino-phényl)-pyrrolidine-3-yl]-heptyldiméthyl-ammonium ; iodure
[1-(4-amino-phényl)-pyrrolidine-3-yl]-octyldiméthyl-ammonium ; iodure
[1-(4-amino-phényl)-pyrrolidine-3-yl]-décyldiméthyl-ammonium ; iodure
[1-(4-amino-phényl)-pyrrolidine-3-yl]-héxadécyldiméthyl-ammonium ; iodure
[1-(4-amino-phényl)-pyrrolidine-3-yl]-hydroxyéthyldiméthyl-ammonium ; chlorure
[1-(4-amino-phényl)-pyrrolidine-3-yl]-hydroxyéthyldiméthyl-ammonium ; iodure.

Plus préférablement on utilisera les composés :
[1-(4-Amino-phényl)-pyrrolidin-3-yl]-triméthyl-ammonium; chlorure
[1-(4-Amino-phényl)-pyrrolidin-3-yl]-diméthyl-tetradécyl-ammonium; bromure
N'-[1-(4-Amino-phényl)-pyrrolidin-3-yl]-N,N-diméthyl-guanidinium chlorure
N-[1-(4-Amino-phényl)-pyrrolidin-3-yl]- guanidinium chlorure
3-[1-(4-Amino-phényl)-pyrrolidin-3-yl]-1-méthyl-3H-imidazol-1-ium; chlorure
[1-(4-Amino-phényl)-pyrrolidin-3-yl]-(2-hydroxy-ethyl)-diméthyl-ammonium; chlorure
[1-(4-Amino-phényl)-pyrrolidin-3-yl]-diméthyl-(3-triméthylsilanyl-propyl)-ammonium ; chlorure
[1-(4-Amino-phényl)-pyrrolidin-3-yl]-(-triméthylammonium-hexyl)-diméthyl-ammonium; dichlorure
1'-(4-Amino-phényl)-1-méthyl-[1,3']bipyrrolidinyl-1-ium; chlorure
3-[1-(4-Amino-phényl)-pyrrolidin-3-yl]-1-(3-triméthylsilanyl-propyl)-3H-imidazol-1-ium ; chlorure
3-[1-(4-Amino-3-méthyl-phényl)-pyrrolidin-3-yl]-1-(3-triméthylsilanyl-propyl)-3H-imidazol-1-ium ; chlorure
[1-(4-amino-phényl)-pyrrolidine-3-yl]-éthyldiméthyl-ammonium ; chlorure
[1-(4-amino-phényl)-pyrrolidine-3-yl]-éthyldiméthyl-ammonium ; iodure
[1-(4-amino-phényl)-pyrrolidine-3-yl]-propyldiméthyl-ammonium ; iodure,
[1-(4-amino-phényl)-pyrrolidine-3-yl]-propyldiméthyl-ammonium ; bromure
[1-(4-amino-phényl)-pyrrolidine-3-yl]-propyldiméthyl-ammonium ; méthosulfate
[1-(4-amino-phényl)-pyrrolidine-3-yl]-butyldiméthyl-ammonium ; iodure
[1-(4-amino-phényl)-pyrrolidine-3-yl]-pentyldiméthyl-ammonium ; iodure
[1-(4-amino-phényl)-pyrrolidine-3-yl]-hexyldiméthyl-ammonium ; iodure
[1-(4-amino-phényl)-pyrrolidine-3-yl]-heptyldiméthyl-ammonium ; iodure
[1-(4-amino-phényl)-pyrrolidine-3-yl]-octyldiméthyl-ammonium ; iodure
[1-(4-amino-phényl)-pyrrolidine-3-yl]-décyldiméthyl-ammonium ; iodure
[1-(4-amino-phényl)-pyrrolidine-3-yl]-hexadécyldiméthyl-ammonium ; iodure
[1-(4-amino-phényl)-pyrrolidine-3-yl]-hydroxyéthyldiméthyl-ammonium ; chlorure
[1-(4-amino-phényl)-pyrrolidine-3-yl]-hydroxyéthyldiméthyl-ammonium ; iodure

Encore plus préférablement, on utilisera les composés suivants :
[1-(4-Amino-phényl)-pyrrolidin-3-yl]-triméthyl-ammonium; chlorure
3-[1-(4-Amino-phényl)-pyrrolidin-3-yl]-1-méthyl-3H-imidazol-1-ium; chlorure
[1-(4-Amino-phényl)-pyrrolidin-3-yl]-(2-hydroxy-ethyl)-diméthyl-ammonium; chlorure
1'-(4-Amino-phényl)-1-méthyl-[1,3']bipyrrolidinyl-1-ium; chlorure, et tout particulièrement les
[1-(4-Amino-phényl)-pyrrolidin-3-yl]-triméthyl-ammonium; chlorure, et
[1-(4-Amino-phényl)-pyrrolidin-3-yl]-(2-hydroxy-ethyl)-diméthyl-ammonium; chlorure.

Le contre ion n'est pas critique quant au résultat de l'invention, tout composé similaire aux composés préférés décrits ci-dessus mais avec un contre ion différent, fait partie intégrante des composés préférés.

Le ou les paraphénylènediamines tertiaires cationiques contenant un noyau pyrrolidinique représentent de 0,001% à 10% et de préférence de 0,005 % à 6 % en poids par rapport au poids total de la composition.

Les composés de formule (I) peuvent être synthétisés selon des méthodes connues et notamment décrites dans la demande WO02/45675.

Les esters de polyols utilisables en tant qu'agents essentiels dans les compositions de l'invention sont en particulier les esters d'acides en C₂-C₃₀ saturés ou insaturés, linéaires ou ramifiés dont la chaîne hydrocarbonée est éventuellement substituée par un ou plusieurs groupements hydroxyles et de polyols en C₂-C₅₀ linéaires, ramifiés ou cycliques éventuellement éthoxylés.

Parmi les acides préférés, on peut citer l'acide laurique, l'acide stéarique, l'acide isostéarique, l'acide oléique, l'acide ricinoléique.

Parmi les polyols, on peut citer l'éthylène glycol, le propylèneglycol, la glycérine, le glucose, le sorbitol ou l'anhydride de sorbitol.

Parmi les esters de polyols utilisables dans les compositions selon l'invention, on peut citer le mono ou distéarate d'éthylène glycol (par exemple CUTINA AGS de COGNIS ou TEGIN EGS de GOLSCHMIDT), l'oléate ou le stéarate de glycéryle (par exemple STEPAN GMO de STEPAN, STEPAN GML de STEPAN, et STEPAN GMS de STEPAN), les lauréates, oléates, stéarates de polyéthylène glycol (par exemple STEPAN PEG 400 ML de STEPAN, STEPAN PEG 200 MO de STEPAN, STEPAN PEG MS de STEPAN).

On préfère les esters de polyols éthoxylés tels que les éthers polyoxyéthylénés des laurates, oléates, stéarates de glycéryle et les éthers polyoxyéthylénés des laurates de sorbitol et/ou d'anhydride de sorbitol (Polysorbates et notamment le polysorbate 20).

Le nombre d'oxyde d'éthylène de ces éthers est alors compris entre 1 et 200 et de préférence de 2 à 100 (exemple AEC PEG 12-glycéryl laurate de A and B CONNOCK, NIKKOL TMGO-10 de NIKKO, AEC PEG-5-Glyceryl Stéarate de A and E CONNOCK, AEC polysorbate 20 de A and E CONNOCK).

Le ou les esters de polyols sont présents dans une concentration allant de 0,01 à 20% de préférence 0,05 à 10% et encore plus préférentiellement de 0,1 à 5% par rapport au poids total de la composition.

Selon un premier mode préféré, la composition selon la présente invention contient en outre au moins un polymère cationique.

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (VI), (VII), (VIII) ou (IX) suivantes : 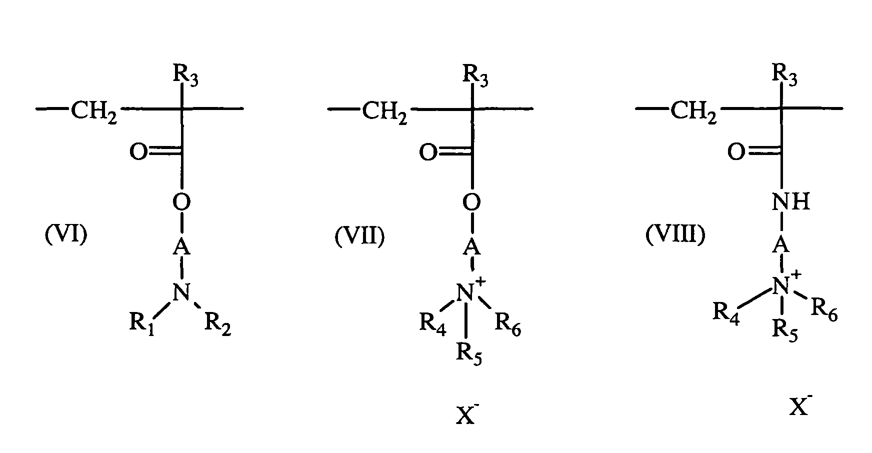 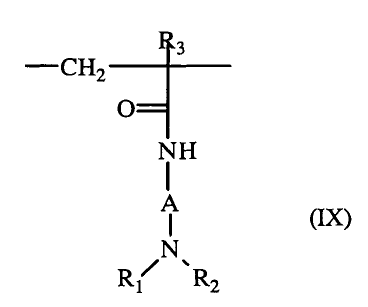 dans lesquelles:
   R₃ désigne un atome d'hydrogène ou un radical CH₃ ;
   A représente un groupe alkyle, linéaire ou ramifié, comprenant de 1 à 6 atomes de carbone, de préférence 2 ou 3 atomes de carbone ou un groupe hydroxyalkyle comprenant de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X⁻ désigne un anion dérivé d'un acide minéral ou organique tel qu'un anion méthosulfate ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymères vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthylaminopropyle quaternisés tel que le produit vendu sous la dénomination "GAFQUAT HS 100" par la société ISP .
(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylamidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (X) ou (XI) : 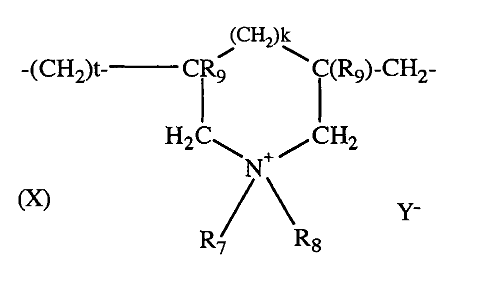 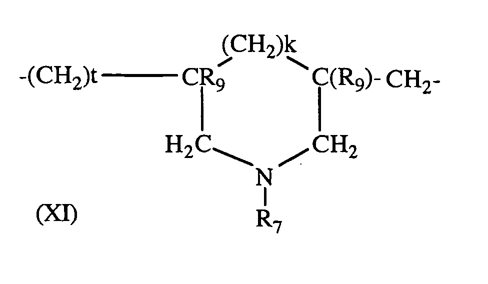 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y- est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(10) Le polymère de diammonium quaternaire contenant des motifs récurrents répondant à la formule : 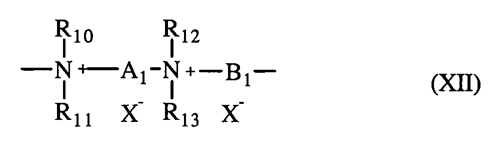 formule (XII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A1, R₁₀ et R₁₂ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement -(CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- .

   De préférence, X- est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (XIII) suivante: 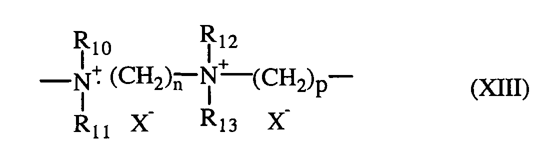 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
(11) Les polymères de polyammonium quaternaire constitués de motifs récurrents de formule (XIV) : 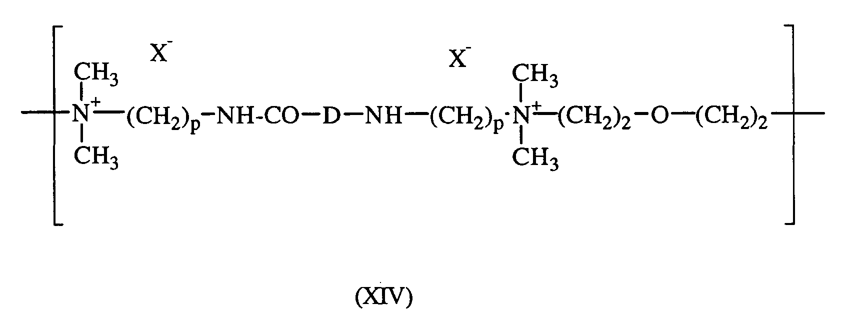 dans laquelle p désigne un nombre entier variant de 1 à 6 environ, D peut être nul ou peut représenter un groupement -(CH₂)ᵣ - CO- dans lequel r désigne un nombre égal à 4 ou à 7, X- est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
(12) Les polymères quaternaires de vinylpyrrolidone et de vinylimidazole tels que par exemple les produits commercialisés sous les dénominations Luviquat FC 905, FC 550 et FC 370 par la société B.A.S.F.
(13) Les polyamines comme le Polyquart H vendu par HENKEL, référencé sous le nom de " POLYETHYLENEGLYCOL (15) TALLOW POLYAMINE " dans le dictionnaire CTFA.
(14) Les polymères réticulés de sels de méthacryloyloxyalkyl(C1-C4) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 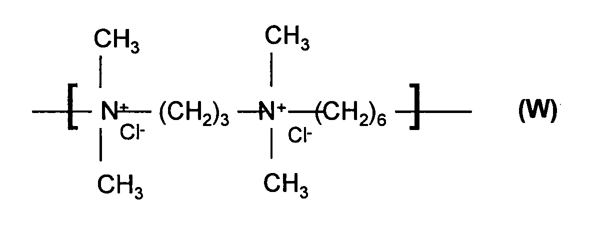 et notamment ceux dont le poids moléculaire, déterminé par chromatographie par perméation de gel, est compris entre 9500 et 9900 ; 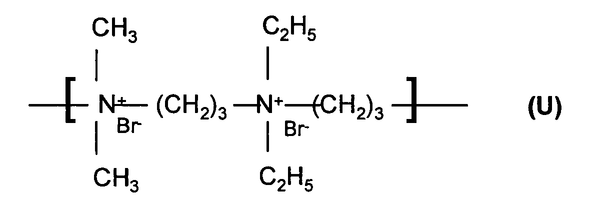 et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

Selon un deuxième mode préféré, la composition selon la présente invention contient en outre au moins un polymère épaississant encore appelé " agents d'ajustement de la rhéologie".

Les agents d'ajustement de la rhéologie peuvent être choisis parmi les amides d'acides gras (diéthanol- ou monoéthanol-amide de coprah, monoéthanolamide d'acide alkyl éther carboxylique oxyéthyléné), les épaississants cellulosiques (hydroxyéthycellulose, hydroxypropylcellulose, carboxyméthylcellulose.), la gomme de guar et ses dérivés (hydroxypropylguar.), les gommes d'origine microbienne (gomme de xanthane, gomme de scléroglucane.), les homopolymères réticulés d'acide acrylique ou d'acide acrylamidopropanesulfonique et les polymères associatifs tels que décrits ci-dessous.

Les polymères associatifs utilisables selon l'invention sont des polymères hydrosolubles capables, dans un milieu aqueux, de s'associer réversiblement entre eux ou avec d'autres molécules.

Leur structure chimique comprend des zones hydrophiles, et des zones hydrophobes caractérisées par au moins une chaîne grasse.

Les polymères associatifs utilisables selon l'invention peuvent être de type anionique, cationique, amphotère et de préférence non ionique.

Leur concentration en poids dans la composition colorante peut varier d'environ 0,01 à 10% du poids total de la composition et dans la composition prête à l'emploi (comprenant l'oxydant) d'environ 0,0025 à 10% du poids total de la composition. Plus préférentiellement, cette quantité varie d'environ 0,1 à 5% en poids dans la composition colorante et d'environ 0,025 à 10% dans la composition prête à l'emploi.

Parmi les polymères associatifs de type anionique, on peut citer :
- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (XV) suivante :

   CH₂ = C R' CH₂ O Bₙ R (XV)

   dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (XV) plus particulièrement préféré est un motif dans lequel R' désigne H, n est égal à 10, et R désigne un radical stéaryl (C₁₈).
   Des polymères associatifs anioniques de ce type sont décrits et préparés, selon un procédé de polymérisation en émulsion, dans le brevet EP-0 216 479.
   Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (XV), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC80® et SALCARE SC90® qui sont des émulsions aqueuses à 30% d'un terpolymère réticulé d'acide méthacrylique, d'acrylate d'éthyle et de steareth-10-allyl éther (40/50/10).
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (XVI) suivante : 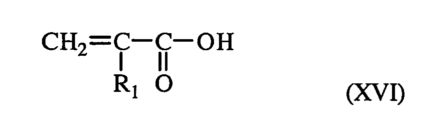 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (XVII) suivante : 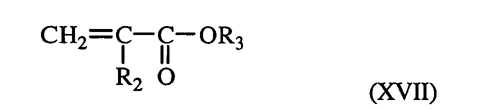 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.
   Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
   Des polymères anioniques de ce type sont par exemple décrits et préparés, selon les brevets US-3 915 921 et 4 509 949.
   Parmi ce type de polymères associatifs anioniques, on utilisera plus particulièrement des polymères formés à partir d'un mélange de monomères comprenant :
   (i) essentiellement de l'acide acrylique,
   (ii) un ester de formule (XVII) décrite ci-dessus et dans laquelle R₂ désigne H ou CH₃, R₃ désignant un radical alkyle ayant de 12 à 22 atomes de carbone, et
   (iii) un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

   Parmi ce type de polymères associatifs anioniques, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précédemment.
   Parmi lesdits polymères ci-dessus, on préfère tout particulièrement selon la présente invention, les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.
- (III) les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608® par la société NEWPHASE TECHNOLOGIES.
- (IV) les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (400E) en dispersion aqueuse à 25%.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄.

A titre d'exemple de ce type de composé on peut citer l'ACULYN 22® vendu par la société ROHM et HAAS, qui est un terpolymère acide méthacrylique/acrylate d'éthyle/méthacrylate de stéaryle oxyalkyléné.

Parmi les polymères associatifs de type cationique, on peut citer :
- (I) les polyuréthanes associatifs cationiques dont la famille a été décrite par la demanderesse dans la demande de brevet français N° 0009609; elle peut être représentée par la formule générale (XVIII) suivante :

   R-X-(P)n-[L-(Y)m]r-L'-(P')p-X'-R' (XVIII)

   dans laquelle :
   R et R', identiques ou différents, représentent un groupement hydrophobe ou un atome d'hydrogène ;
   X et X', identiques ou différents, représentent un groupement comportant une fonction amine portant ou non un groupement hydrophobe, ou encore le groupement L" ;
   L, L' et L", identiques ou différents, représentent un groupement dérivé d'un diisocyanate ;
   P et P', identiques ou différents, représentent un groupement comportant une fonction amine portant ou non un groupement hydrophobe ;
   Y représente un groupement hydrophile ;
   r est un nombre entier compris entre 1 et 100, de préférence entre 1 et 50 et en particulier entre 1 et 25,
   n, m, et p valent chacun indépendamment des autres entre 0 et 1000 ;
   la molécule contenant au moins une fonction amine protonée ou quaternisée et au moins un groupement hydrophobe.
   Dans un mode de réalisation préféré de ces polyuréthanes, les seuls groupements hydrophobes sont les groupes R et R' aux extrémités de chaîne.
   Une famille préférée de polyuréthanes associatifs cationiques est celle correspondant à la formule (XVIII) décrite ci-dessus et dans laquelle :
   R et R' représentent tous les deux indépendamment un groupement hydrophobe,
   X, X' représentent chacun un groupe L",
   n et p valent entre 1 et 1000 et
   L, L', L'', P, P', Y et m ont la signification indiquée ci-dessus.

   Une autre famille préférée de polyuréthanes associatifs cationiques est celle correspondant à la formule (XVIII) ci-dessus dans laquelle :
   R et R' représentent tous les deux indépendamment un groupement hydrophobe, X, X' représentent chacun un groupe L", n et p valent 0, et L, L', L", Y et m ont la signification indiquée ci-dessus.

   Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.
   Encore une autre famille préférée de polyuréthanes associatifs cationiques est celle correspondant à la formule (Ia) ci-dessus dans laquelle :
   R et R' représentent tous les deux indépendamment un groupement hydrophobe,
   X et X' représentent tous les deux indépendamment un groupement comportant une amine quaternaire,
   n et p valent zéro, et
   L, L', Y et m ont la signification indiquée ci-dessus.

   La masse moléculaire moyenne en nombre des polyuréthanes associatifs cationiques est comprise de préférence entre 400 et 500 000, en particulier entre 1000 et 400 000 et idéalement entre 1000 et 300 000.
   Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.
   Préférentiellement, le groupement hydrocarboné provient d'un composé monofonctionnel.
   A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.
   Lorsque X et/ou X' désignent un groupement comportant une amine tertiaire ou quaternaire, X et/ou X' peuvent représenter l'une des formules suivantes : 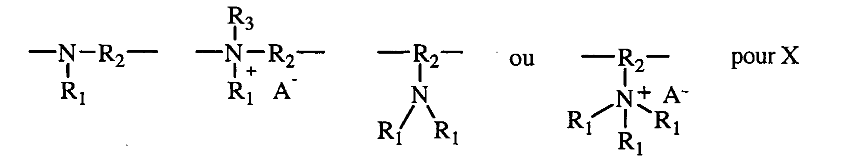 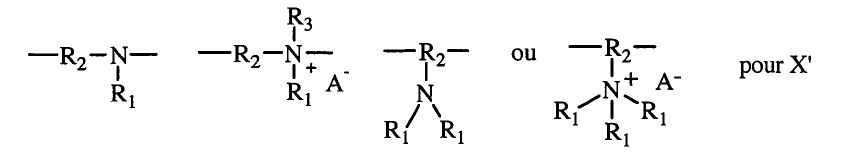 dans lesquelles :
   R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
   R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
   A⁻ est un contre-ion physiologiquement acceptable.

   Les groupements L, L' et L" représentent un groupe de formule : 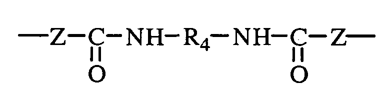 dans laquelle :
   Z représente -O-, -S- ou -NH- ; et
   R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

   Les groupements P et P', comprenant une fonction amine peuvent représenter au moins l'une des formules suivantes : 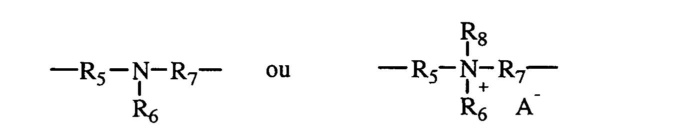 ou 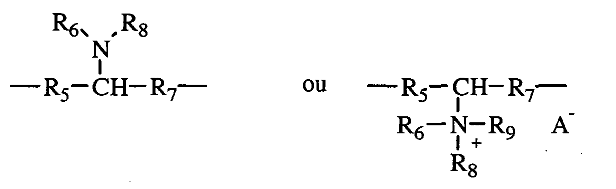 ou 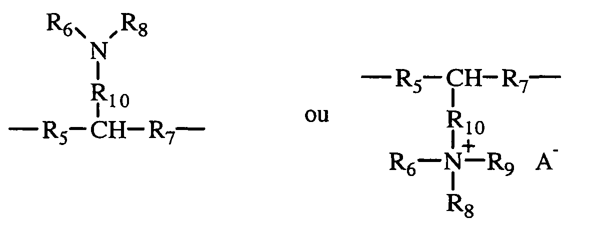 dans lesquelles :
   R₅ et R₇ ont les mêmes significations que R₂ défini précédemment;
   R₆, R₈ et R₉ ont les mêmes significations que R₁ et R₃ définis précédemment ;
   R₁₀ représente un groupe alkylène, linéaire ou ramifié, éventuellement insaturé et pouvant contenir un ou plusieurs hétéroatomes choisis parmi N, O, S et P,
   et A⁻ est un contre-ion physiologiquement acceptable.

   En ce qui concerne la signification de Y, on entend par groupement hydrophile, un groupement hydrosoluble polymérique ou non.
   A titre d'exemple, on peut citer, lorsqu'il ne s'agit pas de polymères, l'éthylèneglycol, le diéthylèneglycol et le propylèneglycol.
   Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).
   Les polyuréthanes associatifs cationiques de formule (XVIII) utilisables selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" utilisable selon la présente invention englobe ces trois types de polymères à savoir les polyuréthanes proprement dits, les polyurées et les polythiourées ainsi que des copolymères de ceux-ci.
   Un premier type de composés entrant dans la préparation du polyuréthane de formule (XVIII) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.
   Comme indiqué précédemment, ce composé peut comporter plus d'un motif à fonction amine. Il s'agit alors d'un polymère portant une répétition du motif à fonction amine.
   Ce type de composés peut être représenté par l'une des formules suivantes :

   HZ-(P)n-ZH,

   ou

   HZ-(P')p-ZH

   dans lesquelles Z, P, P', n et p sont tels que définis plus haut.
   A titre d'exemple de composé à fonction amine, on peut citer la N-méthyldiéthanolamine, la N-tert-butyl-diéthanolamine, la N-sulfoéthyldiéthanolamine.
   Le deuxième composé entrant dans la préparation du polyuréthane de formule (XVIII) est un diisocyanate correspondant à la formule :

   O=C=N-R₄-N=C=O

   dans laquelle R₄ est défini plus haut.
   A titre d'exemple, on peut citer le méthylènediphényldiisocyanate, le méthylènecyclohexanediisocyanate, l'isophoronediisocyanate, le toluènediisocyanate, le naphtalènediisocyanate, le butanediisocyanate, l'hexanediisocyanate.
   Un troisième composé entrant dans la préparation du polyuréthane de formule (XVIII) est un composé hydrophobe destiné à former les groupes hydrophobes terminaux du polymère de formule (XVIII).
   Ce composé est constitué d'un groupe hydrophobe et d'une fonction à hydrogène labile, par exemple une fonction hydroxyle, amine primaire ou secondaire, ou thiol.
   A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné alpha-hydroxyle.
   Le groupe hydrophobe du polyuréthane de formule (XVIII) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.
   Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.
   Les fonctions à hydrogène labile sont des fonctions alcool, amine primaire ou secondaire, ou thiol. Ce composé peut être un polymère terminé aux extrémités des chaînes par l'une de ces fonctions à hydrogène labile.
   A titre d'exemple, on peut citer, lorsqu'il ne s'agit pas de polymères, l'éthylèneglycol, le diéthylèneglycol et le propylèneglycol.
   Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).
   Le groupe hydrophile noté Y dans la formule (XVIII) est facultatif. En effet, les motifs à fonction amine quaternaire ou protonée peuvent suffire à apporter la solubilité ou l'hydrodispersibilité nécessaire pour ce type de polymère dans une solution aqueuse.
   Bien que la présence d'un groupe Y hydrophile soit facultative, on préfère cependant des polyuréthanes associatifs cationiques comportant un tel groupe.
- (II) les dérivés de cellulose quaternisée et les polyacrylates à groupements latéraux aminés non cycliques.
   Les dérivés de cellulose quaternisée sont en particulier,
   - les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
   - les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quaternisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C12) et CRODACEL QS® (alkyle en C₁₈) commercialisés par la société CRODA.

### Polymères associatifs amphotères

Les polymères associatifs amphotères sont choisis de préférence parmi ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Les polymères associatifs amphotères préférés selon l'invention comprennent, ou sont préparés en copolymérisant :
(1) au moins un monomère de formule (XIX) ou (XX) : 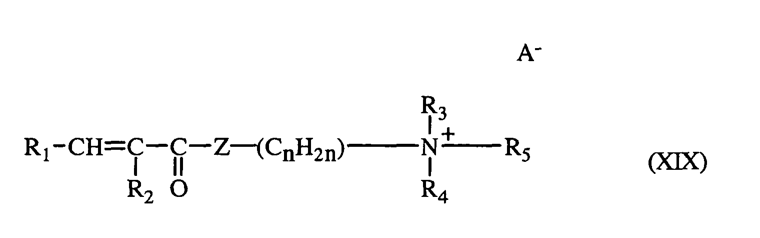 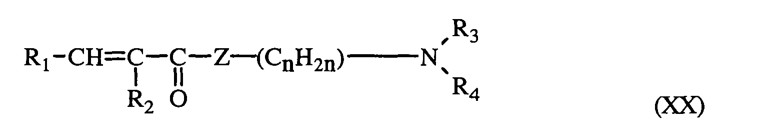 dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A- est un anion issu d'un acide organique ou minéral, tel qu'un anion méthosulfate ou un halogénure tel que chlorure ou bromure;
2) au moins un monomère de formule (XXI)

   R₆-CH=CR₇-COOH (XXI)

   dans laquelle, R₆ et R₇, identiques ou différents, représentent un atome d'hydrogène ou un radical méthyle;
   et
3) au moins un monomère de formule (XXII) :

   R⁶-CH=CR₇-COXR₈ (XXII)

   dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
l'un au moins des monomères de formule (XIX), (XX) ou (XXII) comportant au moins une chaîne grasse.

Les monomères de formule (XIX) et (XX) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
ces monomères étant éventuellement quaternisés, par exemple par un halogénure d'alkyle en C₁-C₄ ou un sulfate de dialkyle en C₁-C₄.

Plus particulièrement, le monomère de formule (XIX) est choisi parmi le chlorure d'acrylamidopropyl triméthyl ammonium et le chlorure de méthacrylamidopropyl triméthyl ammonium.

Les monomères de formule (XXI) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (XXI) est l'acide acrylique.

Les monomères de formule (XXII) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères selon l'invention comprennent de préférence de 1 à 10 % moles du monomère comportant une chaîne grasse (monomère de formule (XIX), (XX) ou (XXII)), et de préférence de 1,5 à 6% moles.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autres monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO9844012.

Parmi les polymères associatifs amphotères selon l'invention, on préfère les terpolymères acide acrylique/chlorure de (méth)acrylamidopropyl triméthyl ammonium/ méthacrylate de stéaryle.

Les polymères associatifs de type non ionique utilisables selon l'invention sont choisis de préférence parmi :
- (1) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- (3) les copolymères de vinyl pyrrolidone et de monomères hydrophobes à chaîne grasse dont on peut citer à titre d'exemple :
   - les produits ANTARON V216® ou GANEX V216® (copolymère vinylpyrrolidone / hexadécène) vendu par la société I.S.P.
   - les produits ANTARON V220® ou GANEX V220® (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- (7) les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂-₁₄ et le produit ELFACOS T212® à chaîne alkyle en C₁₈ de chez AKZO.

Le produit DW 1206B® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Selon un troisième mode préféré, la composition selon la présente invention contient en outre au moins un tensioactif additionnel.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄) aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) additionnels :

Ces agents tensioactifs non ioniques additionnels sont différents des esters de polyols utilisés en tant que composants essentiels dans les compositions selon l'invention.

Les agents tensioactifs non-ioniques additionnels sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine.

### (iii) Tensioactif(s) amphotère(s) ou zwittérionique(s) :

Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO-)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL® C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs additionnels (autres que les esters de polyols) présents dans la composition selon l'invention peuvent varier de 0,01 à 40% et de préférence de 0,5 à 30% du poids total de la composition.

La composition de la présente invention peut en outre comprendre une ou plusieurs bases d'oxydation additionnelles classiquement utilisées en teinture d'oxydation autres que les paraphénylénediamines de formule I. A titre d'exemple, ces bases d'oxydation additionnelles sont choisies parmi les phénylènediamines , les bis-phénylalkylènediamines, les para-aminophénols, les ortho-aminophénols, les bases hétérocycliques autres que les paraphénylènediamines hétérocycliques utiles pour l'invention et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

La ou les bases d'oxydation additionnelles présentes dans la composition de l'invention sont en général présentes en quantité comprise allant de 0,001 à 20 % en poids environ du poids total de la composition tinctoriale, de préférence allant de 0,005 à 6 %.

La composition selon l'invention contient de préférence un ou plusieurs coupleurs additionnels conventionnellement utilisés pour la teinture de fibres kératiniques. Parmi ces coupleurs, on peut notamment citer les métaphénylènediamines, les métadiphénols, les coupleurs naphtaléniques, les coupleurs hétérocycliques ainsi que leur sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en quantité comprise allant de 0,001 à 20 % en poids environ du poids total de la composition tinctoriale, de préférence allant de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques utilisables selon l'invention, on peut citer de manière non limitative les composés suivants :
- 1,4-diamino-2-nitrobenzène,
- 1-amino-2 nitro-4-β-hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoïques cationiques décrits dans les demandes de brevets WO 95/15144, WO-95/01772 et EP-714954 dont le contenu fait partie intégrante de l'invention.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

La composition selon l'invention peut également contenir au moins un solvant hydroxylé, tels que notamment l'éthanol, le propylèneglycol, le glycérol, les monoéthers de polyols, l'alcool benzylique.

Elle peut aussi contenir un solvant non hydroxylé.

Les solvants hydroxylés et les solvants non hydroxylés sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (XXIII) suivante : 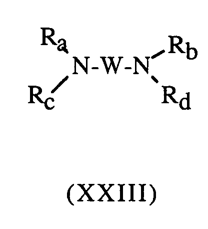 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et qu'on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES DE COMPOSITION SELON L'INVENTION

### Exemple 1 :

### Composition colorante : (exprimée en grammes)

| | |
|---|---|
| Alcool oléique | 6 |
| Acide oléique | 3 |
| Alcool oléique polyglycérolé à 2 moles de glycérol | 6 |
| Alcool oléique polyglycérolé à 6 moles de glycérol | 6 |
| Laurylaminosuccinamate de diéthylaminopropyle, sel de sodium | 3 |
| Amine oléique oxyéthylénée à 2 moles d'oxyde éthylène | 7 |
| Monoéthanolamide d'acide alkyl éther carboxylique à 2 moles d'oxyde d'éthylène | 10 |
| Polysorbate 20 | 2,5 |
| Acétate d'ammonium | 20 |
| propylène glycol | 20 |
| Acide dilinoléique | 1,5 |
| Réducteurs, antioxydants | 0,915 |
| Séquestrants | 1 |
| 1,3-dihydroxybenzène (résorcinol) | 0,085 |
| [1-(4-aminophényl)-pyrrolidin-3-yl]-triméthylammonium, chlorure | 1,0 ' |
| 2-méthyl-5-amino-phénol | 0,5 |
| Niacinamide | 0,2 |
| Parfum | Qs |
| Ammoniaque (à 20,5% en ammoniac) | 10,2 |
| Eau déminéralisée qsp | 100 |

Au moment de l'emploi, on mélange poids pour poids cette composition avec un lait oxydant à 6% de peroxyde d'hydrogène. On applique pendant 30 minutes le mélange obtenu sur des cheveux gris à 90% de blancs. On obtient sur ces cheveux après rinçage, shampooing et séchage une coloration brun violet.

### Exemple 2 :

### Composition colorante : (exprimée en grammes)

| | |
|---|---|
| Alcool oléique | 4 |
| Acide oléique | 5 |
| Alcool oléique polyglycérolé à 2 moles de glycérol | 4 |
| Alcool laurique polyglycérolé à 4 moles de glycérol | 3,6 |
| Amide d'acides de colza oxyéthyléné à 4 moles d'oxyde d'éthylène | 8 |
| Amine oléique oxyéthylénée à 2 moles d'oxyde éthylène | 4 |
| Alcool décylique oxyéthyléné à 3 moles d'oxyde d'éthylène | 2,7 |
| PEG-4 oléate | 2,5 |
| Propylène glycol | 20 |
| Acide adipique | 1,3 |
| Réducteurs, antioxydants | 0,63 |
| Séquestrant | 1 |
| 3-[1-(4-aminophényl)-pyrrolidine-3-yl]-1-méthyl-3H-imidazol-1-ium, chlorure | 0,8 |
| 5N(β-hydroxyéthyl)amino-2-méthyl-phénol) | 0,4 |
| Monoéthanolamine pure | 2 |
| Panthénol | 0,1 |
| Parfum | Qs |
| Ammoniaque (à 20,5% en ammoniac) | 10 |
| Eau déminéralisée qsp | 100 |

Au moment de l'emploi, on mélange poids pour poids cette composition avec un lait oxydant à 6% de peroxyde d'hydrogène. On applique pendant 30 minutes le mélange obtenu sur des cheveux gris à 90% de blancs. On obtient sur ces cheveux après rinçage, shampooing et séchage une coloration violine.

## Revendications

1. Composition tinctoriale pour la teinture des fibres kératiniques comprenant dans un milieu de teinture approprié au moins une paraphénylènediamine tertiaire cationique contenant un noyau pyrrolidinique et au moins un ester de polyol.

2. Composition selon la revendication 1, dans laquelle la paraphénylène tertiaire cationique correspond à la formule 1 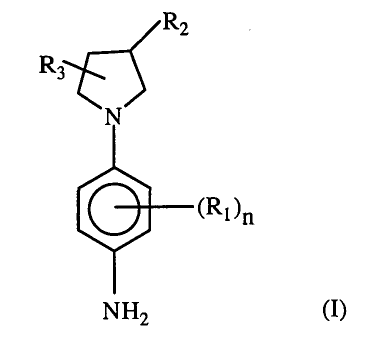 dans laquelle
• n varie de 0 à 4, étant entendu que lorsque n est supérieur ou égal à 2 alors les radicaux R₁ peuvent être identiques ou différents,
• R₁ représente un atome d'halogène ; une chaîne hydrocarbonée en C₁-C₆, aliphatique ou alicyclique, saturée ou insaturée, la chaîne pouvant être interrompue par un ou plusieurs atomes d'oxygène, d'azote, de silicium, de soufre ou par un groupement SO₂, et pouvant être substituée par un ou plusieurs radicaux hydroxy ou amino ; un radical onium Z, le radical R₁ ne comportant pas de liaison peroxyde, ni de radicaux diazo, nitro ou nitroso ,
R₂ représente un radical onium Z ou un radical -X-C=NR₈-NR₉R₁₀ dans lequel X représente un atome d'oxygène ou un radical -NR₁₁ et R₈, R₉, R₁₀ et R₁₁ représentent un atome d'hydrogène, un radical alkyle en C₁-C₄ ou un radical hydroxyalkyle en C₁-C₄,
R₃ représente un atome d'hydrogène ou un radical hydroxy.

3. Composition selon la revendication 2, dans laquelle la paraphénylènediamine tertiaire cationique est telle que n est égal à 0.

4. Composition selon la revendication 2, dans laquelle la paraphénylènediamine tertiaire cationique est telle que n est égal à 1 et R₁ est choisi dans le groupe formé par un atome d'halogène ; une chaîne hydrocarbonée à C₁-C₆, aliphatique ou alicyclique, saturée ou insaturée ; un ou plusieurs atomes de carbone pouvant être remplacés par un atome d'oxygène, d'azote, de silicium, de soufre, par un groupement SO₂, le radical R₁ ne comportant pas de liaison peroxyde, ni de radicaux diazo, nitro ou nitrozo.

5. Composition selon l'une des revendications 2 à 4, dans laquelle la paraphénylèdiamine tertiaire cationique est telle que R₁ est choisi parmi le chlore, le brome, radical alkyle en C₁-C₄, hydroxyalkyle en C₁-C₄, aminoalkyle en C₁-C₄, alcoxy en C₁-C₄, hydroxyalcoxy en C₁-C₄.

6. Composition selon la revendication 5, dans laquelle la paraphénylènediamine tertiaire cationique est telle que R₁ est choisi parmi un radical méthyle, hydroxyméthyle, 2-hydroxyéthyle, 1,2-dihydroxyéthyle, méthoxy, isopropyloxy, 2-hydroxyéthoxy.

7. Composition selon l'une des revendications 2 à 6, dans laquelle la paraphénylènediamine tertiaire cationique est telle que R₂ représente le radical onium Z correspondant à la formule (II) 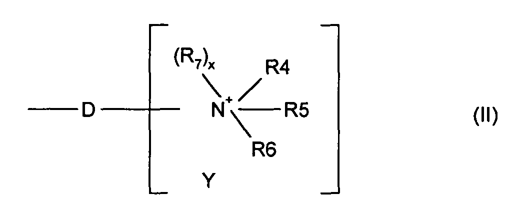 dans laquelle
• D est une simple liaison ou une chaîne alkylène en C₁-C₁₄, linéaire ou ramifiée pouvant être interrompue par un ou plusieurs hétéroatomes choisis parmi l'oxygène, le soufre ou l'azote, et pouvant être substituée par un ou plusieurs radicaux hydroxyle, alcoxy en C₁-C₆ ou amino, et pouvant porter une ou plusieurs fonctions cétone ;
• R₄, R₅ et R₆, pris séparément, représentent un radical alkyle en C₁-C₁₅ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical amidoalkyle en C₁-C₆ ; un radical trialkyl(C₁-C₆)silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl(C₁-C₆)carbonyle, amido ou alkyl(C₁-C₆)sulfonyle ; ou
• R₄, R₅ et R₆ ensemble, deux à deux, forment, avec l'atome d'azote auxquels ils sont rattachés, un cycle saturé carboné à 4, 5, 6 ou 7 chaînons pouvant contenir un ou plusieurs hétéroatomes, le cycle cationique pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical carboxyle, un radical alkyl(C₁-C₆)carbonyle, un radical thio (-SH), un radical thioalkyle (-R-SH) en C₁-C₆, un radical alkyl(C₁-C₆)thio, un radical amino, un radical amino mono ou disubstitué par un radical alkyl(C₁-C₆), alkyl(C₁-C₆)carbonyle, amido ou alkyl(C₁-C₆)sulfonyle ;
• R₇ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆ ; un radical aminoalkyle en C₁-C₆ dont l'amine est mono- ou di-subsituée par un radical alkyl(C₁-C₆), alkyl(C₁-C₆)carbonyle, amido ou alkyl(C₁-C₆)sulfonyle ; un radical carboxyalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆; un radical alkyl(C₁-C₆)sulfonylalkyle en C₁-C₆; un radical alkyl(C₁-C₆)carbonylalkyle en C₁-C₆; un radical N-alkyl(C₁-C₆)carbamylalkyle en C₁-C₆; un radical N-alkyl(C₁-C₆)sulfonamidoalkyle en C₁-C₆;
• x est 0 ou 1,
- lorsque x = 0, alors le bras de liaison est rattaché à l'atome d'azote portant les radicaux R₄ à R₆,
- lorsque x = 1, alors deux des radicaux R₄ à R₆ forment conjointement avec l'atome d'azote auquel ils sont rattachés un cycle saturé à 4, 5, 6 ou 7 chaînons et D est lié à un atome de carbone du cycle saturé ;
• Y est un contre ion.

8. Composition selon la revendication 7, dans laquelle la paraphénylènediamine tertiaire cationique est telle que R₂ correspond à la formule II dans laquelle x est égal à 0 et R₄, R₅ et R₆ séparément sont choisis de préférence parmi un radical alkyle en C₁-C₆, un radical monohydroxyalkyle en C₁-C₄, un radical polyhydroxyalkyle en C₂-C₄, un radical alcoxy(C₁-C₆)alkyle en C₁-C₄, un radical amidoalkyle en C₁-C₆, un radical trialkyl(C₁-C₆)silanealkyle en C₁-C₆, ou R₄ avec R₅ forment ensemble un cycle azétidine, un cycle pyrrolidine, pipéridine, pipérazine, morpholine, R₆ étant choisi dans ce cas parmi un radical alkyle en C₁-C₆; un radical monohydroxyalkyle en C₁-C₆; un radical polyhydroxyalkyle en C₂-C₆; un radical aminoalkyle en C₁-C₆, un radical aminoalkyle mono ou disubstitué par un radical alkyl(C₁-C₆), alkyl(C₁-C₆)carbonyle, amido ou alkyl(C₁-C₆)sulfonyle ; un radical carbamylalkyle en C₁-C₆; un radical trialkyl(C₁-C₆)silanealkyle en C₁-C₆; un radical alkyl(C₁-C₆)carboxyalkyle en C₁-C₆; un radical alkyl(C₁-C₆)carbonyalkyle en C₁-C₆; un radical N-alkyl(C₁-C₆)carbamylalkyle en C₁-C₆.

9. Composition selon la revendication 7, dans laquelle la paraphénylènediamine tertiaire cationique est telle que R₂ correspond à la formule II dans laquelle x est égal à 1 et R₇ est choisi parmi un radical alkyle en C₁-C₆; un radical monohydroxyalkyle en C₁-C₆; un radical polyhydroxyalkyle en C₂-C₆; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, amido ou alkyl(C₁-C₆)sulfonyle ; un radical carbamylalkyle en C₁-C₆; un radical trialkyl(C₁-C₆)silanealkyle en C₁-C₆ ; un radical alkyl(C₁-C₆)carboxyalkyle en C₁-C₆; un radical alkyl(C₁-C₆)carbonylalkyle en C₁-C₆ ; un radical N-alkyl(C₁-C₆)carbamylalkyle en C₁-C₆; R₄ avec R₅ ensemble forment un cycle azétidine, pyrrolidine, pipéridine, pipérazine, morpholine, R₆ étant choisi dans ce cas parmi un radical alkyle en C₁-C₆; un radical monohydroxyalkyle en C₁-C₆; un radical polyhydroxyalkyle en C₂-C₆; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, amido ou alkyl(C₁-C₆)sulfonyle ; un radical carbamylalkyle en C₁-C₆ ; un radical trialkyl(C₁-C₆)silanealkyle en C₁-C₆; un radical alkyl(C₁-C₆)carboxyalkyle en C₁-C₆; un radical alkyl(C₁-C₆)carbonylalkyle en C₁-C₆ ; un radical N-alkyl(C₁-C₆)carbamylalkyle en C₁-C₆.

10. Composition selon l'une des revendications 2 à 9, dans laquelle la paraphénylènediamine tertiaire cationique est telle que D est une simple liaison ou une chaîne alkylène pouvant être substituée.

11. Composition selon l'une des revendications 7 à 10, dans laquelle la paraphénylènediamine tertiaire cationique est telle que R2 est un radical trialkylammonium.

12. Composition selon l'une des revendications 2 à 6, dans laquelle la paraphénylènediamine tertiaire cationique est telle que R₂ représente le radical onium Z correspondant à la formule III 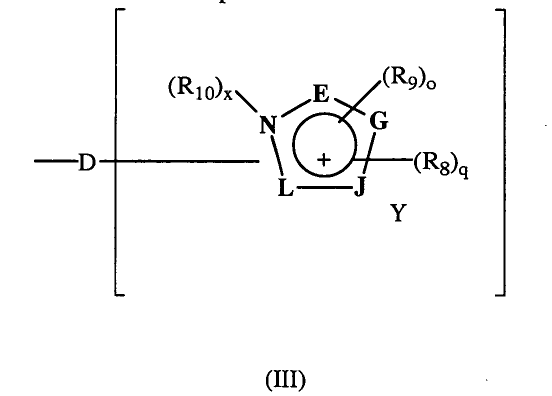 dans laquelle
• D est une simple liaison ou une chaîne alkylène en C₁-C₁₄, linéaire ou ramifiée pouvant être interrompue par un ou plusieurs hétéroatomes choisis parmi l'oxygène, le soufre ou l'azote, et pouvant être substituée par un ou plusieurs radicaux hydroxyle, alcoxy en C₁-C₆ ou amino, et pouvant porter une ou plusieurs fonctions cétone;
• les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique,
• q est un nombre entier compris entre 0 et 4 inclus ;
• o est un nombre entier compris entre 0 et 3 inclus ;
• q+o est un nombre entier compris entre 0 et 4 ;
• les radicaux R₈, identiques ou différents, représentent un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical carboxyle, un radical alkylcarbonyle en C₁-C₆, un radical thio, un radical thioalkyle en C₁-C₆, un radical alkyl(C₁-C₆)thio, un radical amino, un radical amino mono- ou di- substitué par un radical alkyl(C₁-C₆), alkyl(C1-C6)carbonyle, amido, alkyl(C₁-C₆)sulfonyle ; un radical monohydroxyalkyle en C₁-C₆ ou un radical polyhydroxyalkyle en C₂-C₆; étant entendu que les radicaux R₈ sont portés par un atome de carbone,
• les radicaux R₉, identiques ou différents, représentent un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; étant entendu que les radicaux R₉ sont portés par un azote,
• R₁₀ représente un radical alkyle en C₁-C₆; un radical monohydroxyalkyle en C₁-C₆; un radical polyhydroxyalkyle en C₂-C₆; un radical aryle ; un radical benzyle ; un radical aminoalkyle en C₁-C₆, un radical aminoalkyle en C₁-C₆ dont l'amine est substituée par un radical alkyl(C₁-C₆), alkyl(C₁-C₆)carbonyle, amido ou alkyl(C₁-C₆)sulfonyle ; un radical carboxyalkyle en C₁-C₆; un radical carbamylalkyle en C₁-C₆; un radical trifluoroalkyle en C₁-C₆; un radical trialkyl(C₁-C₆)silanealkyle en C₁-C₆; un radical sulfonamidoalkyle en C₁-C₆; un radical alkyl(C₁-C₆)carboxyalkyle en C₁-C₆ ; un radical alkyl(C₁-C₆)sulfinylalkyle en C₁-C₆; un radical alkyl(C₁-C₆)sulfonylalkyle en C₁-C₆ ; un radical alkyl(C₁-C₆)carbonylalkyle en C₁-C₆; un radical N-alkyl(C₁-C₆)carbamylalkyle en C₁-C₆; un radical N-alkyl(C₁-C₆)sulfonamidoalkyle en C₁-C₆;
• x est 0 ou 1
- lorsque x = 0, le bras de liaison D est rattaché à l'atome d'azote,
- lorsque x = 1, le bras de liaison D est rattaché à l'un des sommets E, G, J ou L,
• Y est un contre-ion.

13. Composition selon la revendication 12, dans laquelle la paraphénylènediamine tertiaire cationique est telle que les sommets E, G, J et L forment un cycle imidazolique.

14. Composition selon la revendication 12 ou 13, dans laquelle la paraphénylènediamine tertiaire cationique est telle que x est égal à 0, D est une simple liaison ou une chaîne alkylène pouvant être substituée.

15. Composition selon l'une des revendications 2 à 6, dans laquelle la paraphénylènediamine tertiaire cationique est telle que R₂ représente un radical onium Z correspondant à la formule IV 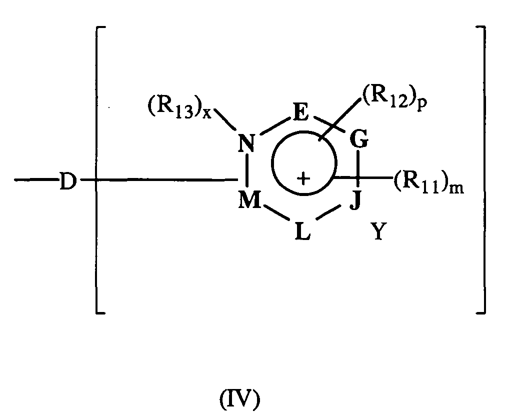 dans laquelle :
• D est une simple liaison ou une chaîne alkylène en C₁-C₁₄, linéaire ou ramifiée pouvant être interrompue par un ou plusieurs hétéroatomes choisis parmi un atome d'oxygène, de soufre ou d'azote, et pouvant être substituée par un ou plusieurs radicaux hydroxyle, alcoxy en C₁-C₆ ou amino, et pouvant porter une ou plusieurs fonctions cétone;
• les sommets E, G, J, L et M identiques ou différents, représentent un atome de carbone, d'oxygène, de soufre ou d'azote pour former un cycle choisi parmi les cycles pyridiniques, pyrimidiniques, pyraziniques, triaziniques et pyridaziniques ;
• p est un nombre entier compris entre 0 et 3 inclus ;
• m est un nombre entier compris entre 0 et 5 inclus ;
• p+m est un nombre entier compris entre 0 et 5 ;
• les radicaux R₁₁, identiques ou différents, représentent un atome d'halogène, un radical hydroxyle, un radical alkyle en C₁-C₆, un radical monohydroxyalkyle en C₁-C₆, un radical polyhydroxyalkyle en C2-C6, un radical alcoxy en C₁-C₆, un radical trialkyl(C₁-C₆)silanealkyle en C₁-C₆, un radical amido, un radical carboxyle, un radical alkylcarbonyle en C₁-C₆, un radical thio, un radical thioalkyle en C₁-C₆, un radical alkyl(C₁-C₆)thio, un radical amino, un radical amino substitué par un radical alkyl(C₁-C₆), alkyl(C₁-C₆)carbonyle, amido ou alkyl(C₁-C₆)sulfonyle ; un radical monohydroxyalkyle en C₁-C₆ ou un radical polyhydroxyalkyle en C₂-C₆; étant entendu que les radicaux R₁₁ sont portés par un atome de carbone,
• les radicaux R₁₂ , identiques ou différents, représentent un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; étant entendu que les radicaux R₁₂ sont portés par un azote,
• R₁₃ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est mono- ou di-substituée par un radical alkyl(C₁-C₆), alkyl(C₁-C₆)carbonyle, amido ou alkyl(C₁-C₆)sulfonyle ; un radical carboxyalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x est 0 ou 1
- lorsque x = 0, le bras de liaison D est rattaché à l'atome d'azote,
- lorsque x = 1, le bras de liaison D est rattaché à l'un des sommets E, G, J, L ou M,
• Y représente un contre ion.

16. Composition selon la revendication 15, dans laquelle les sommets E, G, J, L et M forment avec l'azote du cycle un cycle choisi parmi les cycles pyridiniques et pyrimidiniques.

17. Composition selon l'une des revendications 15 ou 16, dans laquelle la paraphénylènediamine tertiaire cationique est telle que x est égal à 0 et R₁₁ est choisi parmi un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical alkylcarbonyle en C₁-C₆, un radical amino, un radical amino mono- ou di-substitué par un radical alkyl(C₁-C₆), alkyl(C₁-C₆)carbonyle, amido ou alkyl(C₁-C₆)sulfonyle ; un radical monohydroxyalkyle en C₁-C₆ ou un radical polyhydroxyalkyle en C₂-C₆ et R₁₂ est choisi parmi un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆.

18. Composition selon l'une des revendications 15 ou 16, dans laquelle la paraphénylènediamine tertiaire cationique est telle que x est égal à 1 et R₁₃ est choisi parmi un radical alkyle en C₁-C₆ ; un radical monohydroxyalkyle en C₁-C₆ ; un radical polyhydroxyalkyle en C₂-C₆ ; un radical aminoalkyle en C₁-C₆, un radical aminoalkyle en C₁-C₆ dont l'amine est mono- ou di- substituée par un radical alkyl(C₁-C₆), alkyl(C₁-C₆)carbonyle, un radical amido ou un radical alkyl(C₁-C₆)sulfonyle ; un radical carbamylalkyle en C₁-C₆ ; un radical trialkyl(C₁-C₆)silanealkyle en C₁-C₆ ; un radical alkyl(C₁-C₆)carbonylalkyle en C₁-C₆ ; un radical N-alkyl(C₁-C₆)carbamylalkyle en C₁-C₆ ; R₁₁ est choisi parmi un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical alkylcarbonyle en C₁-C₆, un radical amino, un radical amino mono- ou di- substitué par un radical alkyl(C₁-C₆), alkyl(C₁-C₆)carbonyle, amido ou alkyl(C₁-C₆)sulfonyle ; et R₁₂ est choisi parmi un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆.

19. Composition selon l'une quelconque des revendications 15 à 18, dans laquelle la paraphénylènediamine tertiaire cationique est telle que R₁₁, R₁₂ et R₁₃ sont des radicaux alkyles pouvant être substitués.

20. Composition selon l'une des revendications 2 à 6, dans laquelle la paraphénylènediamine tertiaire cationique est telle que le radical R₂ est radical de formule -XP(O)(O-)OCH₂CH₂N⁺(CH₃)₃ où X représente un atome d'oxygène ou un radical-NR₁₄, R₁₄ représentant un hydrogène, un radical alkyle en C₁-C₄ ou un radical hydroxyalkyle.

21. Composition selon l'une des revendications 2 à 6, dans laquelle la paraphénylènediamine tertiaire cationique est telle que le radical R₂ est un radical guanidine de formule -X-C=NR₈-NR₉R₁₀, X représente un atome d'oxygène ou un radical -NR₁₁, R₈, R₉, R₁₀ et R₁₁ représentant un hydrogène, un radical alkyle en C₁-C₄ ou un radical hydroxyalkyle.

22. Composition selon l'une quelconque des revendications précédentes, dans laquelle la paraphénylène tertiaire cationique est choisie dans le groupe formé par les
[1-(4-Amino-phényl)-pyrrolidin-3-yl]-triméthyl-ammonium; chlorure,
[1-(4-Amino-phényl)-pyrrolidin-3-yl]-diméthyl-tetradécyl-ammonium; bromure
N'-[1-(4-Amino-phényl)-pyrrolidin-3-yl]-N,N-diméthyl-guanidinium chlorure
N-[1-(4-Amino-phényl)-pyrrolidin-3-yl]- guanidinium chlorure
3-[1-(4-Amino-phényl)-pyrrolidin-3-yl]-1-méthyl-3H-imidazol-1-ium; chlorure
[1-(4-Amino-phényl)-pyrrolidin-3-yl]-(2-hydroxy-ethyl)-diméthyl-ammonium; chlorure
[1-(4-Amino-phényl)-pyrrolidin-3-yl]-diméthyl-(3-triméthylsilanyl-propyl)-ammonium ; chlorure
[1-(4-Amino-phényl)-pyrrolidin-3-yl]-(-triméthylammonium-hexyl)-diméthyl-ammonium; dichlorure
[1-(4-Amino-phényl)-pyrrolidin-3-yl]-oxophosphorylcholine
{2-[1-(4-Amino-phényl)-pyrrolidin-3-yloxy]-ethyl}-triméthyl-ammonium; chlorure
1-{2-[1-(4-Amino-phényl)-pyrrolidin-3-yloxy]-ethyl}-1-méthyl-pyrrolidinium; chlorure
3-{3-[1-(4-Amino-phényl)-pyrrolidin-3-yloxy]-propyl}-1-méthyl-3H-imidazol-1-ium ; chlorure
1-{2-[1-(4-Amino-phényl)-pyrrolidin-3-yloxy]-ethyl}-1-méthyl-piperidinium; chlorure
3-{3-[1-(5-triméthylsilanylethyl-4-Amino-3-triméthylsilanylethyl -phényl)-pyrrolidin-3-yloxy]-propyl}-1-méthyl-3H-imidazol-1-um ; chlorure
[1-(4-Amino-3-méthyl-phényl)-pyrrolidin-3-yl]-triméthyl-ammonium; chlorure
[1-(4-Amino-3-méthyl-phényl)-pyrrolidin-3-yl]-diméthyl-tetradecyl-ammonium; chlorure
N'-[1-(4-Amino-3-méthyl-phényl)-pyrrolidin-3-yl]-N,N-diméthyl- guanidinium chlorure
N-[1-(4-Amino-3-méthyl-phényl)-pyrrolidin-3-yl]- guanidinium chlorure
3-[1-(4-Amino-3-méthyl-phényl)-pyrrolidin-3-yl]-1-méthyl-3H-imidazol-1-ium; chlorure
[1-(4-Amino-3-méthyl-phényl)-pyrrolidin-3-yl]-(2-hydroxy-ethyl)-diméthyl-ammonium chlorure
[1-(4-Amino-3-méthyl-phényl)-pyrrolidin-3-yl]-diméthyl-(3-triméthylsilanyl-propyl ammonium ; chlorure
[1-(4-Amino-3-méthyl-phényl)-pyrrolidin-3-yl]-(-triméthylammonium-hexyl)-diméthyl-ammonium dichlorure
[1-(4-Amino-3-méthyl-phényl)-pyrrolidin-3-yl]-oxophosphorylcholine
{2-[1-(4-Amino-3-méthyl-phényl)-pyrrolidin-3-yloxy]-ethyl}-triméthyl-ammonium; chlorure
1-{2-[ 1-(4-Amino-3-méthyl-phényl)-pyrrolidin-3-yloxy]-ethyl}-1-méthyl-pyrrolidinium ; chlorure
3-{3-[1-(4-Amino-3-méthyl-phényl)-pyrrolidin-3-yloxy]-propyl}-1-méthyl-3H-imidazol-1-um ; chlorure
1-{2-[1-(4-Amino-3-méthyl-phényl)-pyrrolidin-3-yloxy]-ethyl}-1-méthyl-piperidinium; chlorure
[1-(4-Amino-3-triméthylsilanylethyl-phényl)-pyrrolidin-3-yl]-triméthyl-ammonium; chlorure
3-[1-(4-Amino-3-triméthylsilanylethyl-phényl)-pyrrolidin-3-yl]-1-méthyl-3H-imidazol-1-ium; chlorure
3-{3-[1-(4-Amino-3- triméthylsilanylethyl -phényl)-pyrrolidin-3-yloxy]-propyl}-1-méthyl-3H-imidazol-1-um ; chlorure
[1-(5-triméthylsilanylethyl-4-Amino-3-triméthylsilanylethyl-phényl)-pyrrolidin-3-yl]-triméthyl-ammonium; chlorure
3-[1-(5-triméthylsilanylethyl-4-Amino-3-triméthylsilanylethyl-phényl)-pyrrolidin-3-yl]-1-méthyl-3H-imidazol-1-ium; chlorure
1'-(4-Amino-phényl)-1-méthyl-[1,3']bipyrrolidinyl-1-ium; chlorure
1'-(4-Amino-3-méthyl-phényl)-1-méthyl-[1,3']bipyrrolidinyl-1-ium; chlorure
3-{[1-(4-Amino-phényl)-pyrrolidin-3-ylcarbamoyl]-méthyl}-1-méthyl-3H-imidazol-1-ium ; chlorure
3-{[1-(4-Amino-3-méthyl-phényl)-pyrrolidin-3-ylcarbamoyl]-méthyl}-1-méthyl-3H- imidazol-1-ium ; chlorure
3-[1-(4-Amino-phényl)-pyrrolidin-3-yl]-1-(3-triméthylsilanyl-propyl)-3H-imidazol-1-ium ; chlorure
3-[1-(4-Amino-phényl)-pyrrolidin-3-yl]-1-(3-triméthylsilanyl-propyl)-3H-imidazol-1-ium ; chlorure
[1-(4-amino-phényl)-pyrrolidine-3-yl]-éthyldiméthyl-ammonium ; chlorure
[1-(4-amino-phényl)-pyrrolidine-3-yl]-éthyldiméthyl-ammonium; iodure
[1-(4-amino-phényl)-pyrrolidine-3-yl]-propyldiméthyl-ammonium ; iodure,
[1-(4-amino-phényl)-pyrrolidine-3-yl]-propyldiméthyl-ammonium ; bromure
[1-(4-amino-phényl)-pyrrolidine-3-yl]-propyldiméthyl-ammonium ; méthosulfate
[1-(4-amino-phényl)-pyrrolidine-3-yl]-butyldiméthyl-ammonium; iodure
[1-(4-amino-phényl)-pyrrolidine-3-yl]-pentyldiméthyl-ammonium ; iodure
[1-(4-amino-phényl)-pyrrolidine-3-yl]-hexyldiméthyl-ammonium; iodure
[1-(4-amino-phényl)-pyrrolidine-3-yl]-heptyldiméthyl-ammonium ; iodure
[1-(4-amino-phényl)-pyrrolidine-3-yl]-octyldiméthyl-ammonium ; iodure
[1-(4-amino-phényl)-pyrrolidine-3-yl]-décyldiméthyl-ammonium ; iodure
[1-(4-amino-phényl)-pyrrolidine-3-yl]-hexadécyldiméthyl-ammonium ; iodure
[1-(4-amino-phényl)-pyrrolidine-3-yl]-hydroxyéthyldiméthyl-ammonium ; chlorure
[1-(4-amino-phényl)-pyrrolidine-3-yl]-hydroxyéthyldiméthyl-ammonium ; iodure.

23. Composition selon l'une des revendications précédentes, dans laquelle la paraphénylène tertiaire cationique est choisie dans le groupe formé par [1-(4-Amino-phényl)-pyrrolidin-3-yl]-triméthyl-ammonium; chlorure ;
[1-(4-Amino-phényl)-pyrrolidin-3-yl]-diméthyl-tetradécyl-ammonium; bromure ;
N'-[1-(4-Amino-phényl)-pyrrolidin-3-yl]-N,N-diméthyl-guanidinium chlorure
N-[1-(4-Amino-phényl)-pyrrolidin-3-yl]- guanidinium chlorure
3-[1-(4-Amino-phényl)-pyrrolidin-3-yl]-1-méthyl-3H-imidazol-1-ium; chlorure ;
[1-(4-Amino-phényl)-pyrrolidin-3-yl]-(2-hydroxy-ethyl)-diméthyl-ammonium; chlorure
[1-(4-Amino-phényl)-pyrrolidin-3-yl]-diméthyl-(3-triméthylsilanyl-propyl)-ammonium ; chlorure ;
[1-(4-Amino-3-méthyl-phényl)-pyrrolidin-3-yl]-triméthyl-ammonium; chlorure
[1-(4-Amino-3-méthyl-phényl)-pyrrolidin-3-yl]-diméthyl-tetradecyl-ammonium; chlorure
N'-[1-(4-Amino-3-méthyl-phényl)-pyrrolidin-3-yl]-N,N-diméthyl- guanidinium chlorure
N-[1-(4-Amino-3-méthyl-phényl)-pyrrolidin-3-yl]- guanidinium chlorure
3-[1-(4-Amino-3-méthyl-phényl)-pyrrolidin-3-yl]-1-méthyl-3H-imidazol-1-ium; chlorure
[1-(4-Amino-3-méthyl-phényl)-pyrrolidin-3-yl]-(2-hydroxy-ethyl)-diméthyl-ammonium chlorure
[1-(4-Amino-3-méthyl-phényl)-pyrrolidin-3-yl]-diméthyl-(3-triméthylsilanyl-propyl ammonium ; chlorure
1'-(4-Amino-phényl)-1-méthyl-[1,3']bipyrrolidinyl-1-ium; chlorure
1'-(4-Amino-3-méthyl-phényl)-1-méthyl-[1,3']bipyrrolidinyl-1-ium; chlorure
3-{[1-(4-Amino-phényl)-pyrrolidin-3-ylcarbamoyl]-méthyl}-1-méthyl-3H-imidazol-1-ium ; chlorure
3-{[1-(4-Amino-3-méthyl-phényl)-pyrrolidin-3-ylcarbamoyl]-méthyl}-1-méthyl-3H- imidazol-1-ium ; chlorure
3-[1-(4-Amino-phényl)-pyrrolidin-3-yl]-1-(3-triméthylsilanyl-propyl)-3H-imidazol-1-ium ; chlorure
3-[1-(4-Amino-3-méthyl-phényl)-pyrrolidin-3-yl]-1-(3-triméthylsilanyl-propyl)-3H-imidazol-1-ium ; chlorure
[1-(4-amino-phényl)-pyrrolidine-3-yl]-éthyldiméthyl-ammonium ; chlorure
[1-(4-amino-phényl)-pyrrolidine-3-yl]-éthyldiméthyl-ammonium ; iodure
[1-(4-amino-phényl)-pyrrolidine-3-yl]-propyldiméthyl-ammonium ; iodure,
[1-(4-amino-phényl)-pyrrolidine-3-yl]-propyldiméthyl-ammonium ; bromure
[1-(4-amino-phényl)-pyrrolidine-3-yl]-propyldiméthyl-ammonium ; méthosulfate
[1-(4-amino-phényl)-pyrrolidine-3-yl]-butyldiméthyl-ammonium ; iodure
[1-(4-amino-phényl)-pyrrolidine-3-yl]-pentyldiméthyl-ammonium ; iodure
[1-(4-amino-phényl)-pyrrolidine-3-yl]-hexyldiméthyl-ammonium ; iodure
[1-(4-amino-phényl)-pyrrolidine-3-yl]-heptyldiméthyl-ammonium ; iodure
[1-(4-amino-phényl)-pyrrolidine-3-yl]-octyldiméthyl-ammonium ; iodure
[1-(4-amino-phényl)-pyrrolidine-3-yl]-décyldiméthyl-ammonium ; iodure
[1-(4-amino-phényl)-pyrrolidine-3-yl]-hexadécyldiméthyl-ammonium ; iodure
[1-(4-amino-phényl)-pyrrolidine-3-yl]-hydroxyéthyldiméthyl-ammonium ; chlorure
[1-(4-amino-phényl)-pyrrolidine-3-yl]-hydroxyéthyldiméthyl-ammonium ; iodure.

24. Composition selon l'une quelconque des revendications précédentes, dans laquelle la paraphénylène tertiaire cationique est choisie dans le groupe formé par [1-(4-Amino-phényl)-pyrrolidin-3-yl]-triméthyl-ammonium; chlorure
[1-(4-Amino-phényl)-pyrrolidin-3-yl]-diméthyl-tetradécyl-ammonium; bromure
N'-[1-(4-Amino-phényl)-pyrrolidin-3-yl]-N,N-diméthyl-guanidinium chlorure
N-[1-(4-Amino-phényl)-pyrrolidin-3-yl]- guanidinium chlorure
3-[1-(4-Amino-phényl)-pyrrolidin-3-yl]-1-méthyl-3H-imidazol-1-ium; chlorure
[1-(4-Amino-phényl)-pyrrolidin-3-yl]-(2-hydroxy-ethyl)-diméthyl-ammonium; chlorure
[1-(4-Amino-phényl)-pyrrolidin-3-yl]-diméthyl-(3-triméthylsilanyl-propyl)-ammonium ; chlorure
[1-(4-Amino-phényl)-pyrrolidin-3-yl]-(-triméthylammonium-hexyl)-diméthyl-ammonium; dichlorure
1'-(4-Amino-phényl)-1-méthyl-[1,3']bipyrrolidinyl-1-ium; chlorure
3-[1-(4-Amino-phényl)-pyrrolidin-3-yl]-1-(3-triméthylsilanyl-propyl)-3H-imidazol-1-ium ; chlorure
3-[1-(4-Amino-3-méthyl-phényl)-pyrrolidin-3-yl]-1-(3-triméthylsilanyl-propyl)-3H-imidazol-1-ium ; chlorure
[1-(4-amino-phényl)-pyrrolidine-3-yl]-éthyldiméthyl-ammonium ; chlorure
[1-(4-amino-phényl)-pyrrolidine-3-yl]-éthyldiméthyl-ammonium ; iodure
[1-(4-amino-phényl)-pyrrolidine-3-yl]-propyldiméthyl-ammonium ; iodure,
[1-(4-amino-phényl)-pyrrolidine-3-yl]-propyldiméthyl-ammonium ; bromure
[1-(4-amino-phényl)-pyrrolidine-3-yl]-propyldiméthyl-ammonium ; méthosulfate
[1-(4-amino-phényl)-pyrrolidine-3-yl]-butyldiméthyl-ammonium ; iodure
[1-(4-amino-phényl)-pyrrolidine-3-yl]-pentyldiméthyl-ammonium ; iodure
[1-(4-amino-phényl)-pyrrolidine-3-yl]-hexyldiméthyl-ammonium ; iodure
[1-(4-amino-phényl)-pyrrolidine-3-yl]-heptyldiméthyl-ammonium ; iodure
[1-(4-amino-phényl)-pyrrolidine-3-yl]-octyldiméthyl-ammonium ; iodure
[1-(4-amino-phényl)-pyrrolidine-3-yl]-décyldiméthyl-ammonium ; iodure
[1-(4-amino-phényl)-pyrrolidine-3-yl]-hexadécyldiméthyl-ammonium ; iodure
[1-(4-amino-phényl)-pyrrolidine-3-yl]-hydroxyéthyldiméthyl-ammonium ; chlorure
[1-(4-amino-phényl)-pyrrolidine-3-yl]-hydroxyéthyldiméthyl-ammonium ; iodure.

25. Composition selon l'une quelconque des revendications précédentes, dans laquelle la paraphénylène tertiaire cationique est choisie dans le groupe formé par les
[1-(4-Amino-phényl)-pyrrolidin-3-yl]-triméthyl-ammonium; chlorure
3-[1-(4-Amino-phényl)-pyrrolidin-3-yl]-1-méthyl-3H-imidazol-1-ium; chlorure
[1-(4-Amino-phényl)-pyrrolidin-3-yl]-(2-hydroxy-ethyl)-diméthyl-ammonium; chlorure
1'-(4-Amino-phényl)-1-méthyl-[1,3']bipyrrolidinyl-1-ium; chlorure.

26. Composition selon l'une quelconque des revendications précédentes, dans laquelle la paraphénylène tertiaire cationique est choisie dans le groupe formé par les
[1-(4-Amino-phényl)-pyrrolidin-3-yl]-triméthyl-ammonium; chlorure, et [1-(4-Amino-phényl)-pyrrolidin-3-yl]-(2-hydroxy-éthyl)-diméthyl-ammonium; chlorure.

27. Composition selon l'une des revendications précédentes, dans laquelle l'ester de polyol est un ester d'acide en C₂-C₃₀ saturé ou insaturé, linéaire ou ramifié dont la chaîne hydrocarbonée est éventuellement substituée par un ou plusieurs groupements hydroxyles et d'un polyol en C₂-C₅₀ linéaire, ramifié ou cyclique éventuellement éthoxylé.

28. Composition selon la revendication 27 telle que l'ester de polyol est un ester d'un acide choisi parmi l'acide laurique, l'acide stéarique, l'acide isostéarique, l'acide oléique, l'acide ricinoléique.

29. Composition selon la revendication 27 ou 28 telle que l'ester de polyol est tel que le polyol est choisi parmi l'éthylène glycol, le propylèneglycol, la glycérine, le glucose, le sorbitol ou l'anhydride de sorbitol.

30. Composition selon l'une des revendications 27 à 29 telle que l'ester de polyol est choisi parmi le mono ou distéarate d'éthylène glycol, l'oléate ou le stéarate de glycéryle, le lauréate de polyéthylène glycol, l'oléate de polyéthylèneglycol et le stéarate de polyéthylène glycol.

31. Composition selon l'une des revendications 27 à 29 telle que l'ester de polyol est un ester de polyol éthoxylé choisi parmi les éthers polyoxyéthylénés des laurates, oléates et stéarates de glycéryle et les éthers polyoxyéthylénés des laurates de sorbitol et/ou d'anhydride de sorbitol.

32. Composition selon la revendication 31 telle que le nombre d'oxyde d'éthylène des esters de polyols est compris entre 1 et 200 et de préférence entre 2 et 100.

33. Composition selon l'une des revendications précédentes telle que le ou les ester(s) de polyol(s) sont présents dans une concentration allant de 0,01 à 20% de préférence de 0,05 à 10%.

34. Composition selon la revendication 33 telle que le ou les ester(s) de polyol(s) sont présents dans une concentration allant de 0,1 à 5% en poids par rapport au poids total de la composition.

35. Composition selon l'une des revendications précédentes dans laquelle la ou les paraphénylènes tertiaires cationiques contenant un noyau pyrrolidinique représente de 0,01 à 10% et de préférence de 0,05 à 6% en poids par rapport au poids total de la composition.

36. Composition selon l'une des revendications précédentes telle qu'elle contient en outre au moins un polymère cationique.

37. Composition selon l'une des revendications précédentes telle qu'elle contient en outre au moins un polymère épaississant.

38. Composition selon l'une des revendications précédentes telle qu'elle contient en outre au moins un tensioactif additionnel choisi dans le groupe formé par les tensioactifs anioniques, les tensioactifs amphotères ou zwittérioniques, les tensioactifs non ioniques autres que les esters de polyols et les tensioactifs cationiques.

39. Composition selon l'une des revendications précédentes telle qu'elle comprend au moins une base d'oxydation additionnelle autre que les paraphénylènediamines tertiaires cationiques à noyau pyrrolidinique choisie parmi les paraphénylènediamines, les bis-phénylalkylènediamines, les paraaminophénols, les orthoaminophénols, les bases hétérocycliques et leurs sels d'addition.

40. Composition selon la revendication 39, dans laquelle la ou les bases d'oxydation additionnelles sont présentes en une quantité comprise entre 0,001 à 20 % en poids et de préférence entre 0,005 et 6 % en poids par rapport au poids total de la composition.

41. Composition selon l'une des revendications précédentes telle qu'elle comprend en outre au moins un coupleur choisi parmi les métaphénylènediamines, les métaaminophénols, les métadiphénols, les coupleurs naphtaléniques, les coupleurs hétérocycliques et leurs sels d'addition.

42. Composition selon la revendication 41 telle que le coupleur choisi parmi le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

43. Composition selon la revendication 41 ou 42, telle que le ou les coupleurs sont présents en une quantité comprise entre 0,001 et 20 % de préférence entre 0,005 et 6 % en poids par rapport au poids total de la composition.

44. Composition selon l'une des revendications précédentes telle qu'elle comprend en outre au moins un colorant direct.

45. Composition selon l'une des revendications précédentes telle qu'elle comprend en outre au moins un solvant hydroxylé tel que l'éthanol, le propylène glycol, le glycérol, les mono- éthers de polyol.

46. Composition selon l'une des revendications précédentes telle qu'elle contient un agent oxydant choisi parmi le peroxyde d'hydrogène, le peroxyde d'urée, les bromates de métaux alcalins, les persels, les peracides et les enzymes oxydases, et de préférence le peroxyde d'hydrogène.

47. Procédé de teinture d'oxydation des fibres kératiniques, **caractérisé en ce qu'**on applique sur les fibres une composition tinctoriale telle que définie dans l'une quelconque des revendications 1 à 45 en présence d'un agent oxydant.

48. Dispositif à plusieurs compartiments dans lequel le premier compartiment contient une composition tinctoriale pour la teinture des fibres kératiniques telle que définie à l'une quelconque des revendications 1 à 45 et un deuxième compartiment contient un agent oxydant.
